# EUROPEAN PATENT APPLICATION

(11) **EP 4 345 091 A1**
(43) Date of publication of application: **03.04.2024**
(21) Application number: 22199251.4
(22) Date of filing: 30.09.2022
(51) Int. Cl.: C07C 233/65, C07C 233/66, A61K 31/166, A61P 31/18

(54) **BENZOIC ACID DERIVATIVES, METHODS AND USES THEREOF**

(30) Priority: 29.09.2022 PT 2022118234
(71) Applicant: Faculdade de Farmácia da Universidade de Lisboa, 1649-003 Lisboa (PT)
(72) Inventor: VICENTE CONSTANTINO, LUIS FILIPE, 1649-003 LISBOA (PT); ALMEIDA PAIS, JOÃO PEDRO, 1700-348 LISBOA (PT); DUARTE DELGADO, TIAGO ALEXANDRE, 2705-293 COLARES (PT); ANTONIUK, OLHA, 3025-041 COIMBRA (PT); RIBEIRO DOS SANTOS ANES, ELSA MARIA, 1649-003 LISBOA (PT); RODRIGUES PIRES, DAVID ALEXANDRE, 2790-361 QUEIJAS (PT)
(74) Representative: Patentree

(57) **Abstract**

The present disclosure relates to novel benzoic acid derivatives, specifically esters and benzamides, and their obtention. Furthermore, the present disclosure relates to the use of such compounds in medicine or veterinary, specifically, in the prevention or treatment of tuberculosis.

## Description

### TECHNICAL FIELD

The present disclosure relates to novel benzoic acid derivatives, specifically esters and benzamides, and their obtention. Furthermore, the present disclosure relates to the use of such compounds in medicine or veterinary, specifically, in the prevention or treatment of tuberculosis.

### BACKGROUND

Decades ago, it was expected that powerful antibiotics developed during the second part of the 20th century would by now have wiped out tuberculosis (TB). On the contrary, virulent and deadly new strains have evolved in the last 20 years and the current situation is far from controlled. After the COVID -19 pandemic began, the number of deaths from tuberculosis that was slowly reducing, started increasing again. In 2020 the number is deaths estimated by WHO was 1.5 million and WHO modelling projections suggest the number of people dying from the disease could be much higher in 2021 and 2022 [1].

One of the problems in treating tuberculosis is due to multi-drug resistant TB (MDR-TB), a form of the disease that is extremely difficult to treat and is regarded as a death sentence in many developing countries. Treatment of MDR-TB remains a challenge as it relies on prolonged second-line drug treatments that are less effective, more toxic, and much more expensive than first-line treatments. [2]. This MDR-TB treatment regimens are too expensive and complicated for poor countries, and even for more advanced health systems. Only about one in three people with drug resistant TB accessed treatment in 2020. [1].

Alternatives to treat this disease are urgently needed. Despite the recent developments, it is still urgent and important to develop compounds for the prevention and treatment of tuberculosis, with improved activity and stability.

These facts are disclosed in order to illustrate the technical problem addressed by the present disclosure.

### GENERAL DESCRIPTION

The present disclosure relates to the development of a library of compounds, composed of novel benzoic acid derivatives, specifically benzamides and esters, with improved stability and activity in the treatment of tuberculosis.

An aspect of the present disclosure relates to the synthesis of amide derivatives as antituberculosis agents. It was surprisingly found that the amides obtained proved to be more active than the esters. The amides obtained proved to be stable in mycobacterial homogenate and are not activated to the corresponding acid, which led us to propose that the amides act as drugs and not as prodrugs.

Based on the International Union of Pure and Applied Chemistry (IUPAC) definitions, an alkyl group is defined as a univalent group derived from alkanes by removal of a hydrogen atom from any carbon atom -CnH₂ₙ₊₁. The groups derived by removal of a hydrogen atom from a terminal carbon atom of unbranched alkanes form a subclass of normal alkyl (n-alkyl) groups H (CH₂)ₙ. The groups RCH₂, R₂CH (R ≠ H), and R₃C (R ≠ H) are primary, secondary and tertiary alkyl groups, respectively.

"Alkyl" includes "lower alkyl" and extends to cover carbon fragments having up to 30 carbon atoms. Examples of alkyl groups include octyl, nonyl, norbornyl, undecyl, dodecyl, tridecyl, tetradecyl, pentadecyl, eicosyl, 3,7-diethyl-2,2-dimethyl-4 -propylnonyl, 2-(cyclododecyl)ethyl, adamantyl, and the like.

"Lower alkyl" means alkyl groups of from 1 to 7 carbon atoms. Examples of lower alkyl groups include methyl, ethyl, propyl, isopropyl, butyl, sec- and tert-butyl, pentyl, hexyl, heptyl, cyclopropyl, cyclobutyl, cyclopentyl, cyclohexyl, cycloheptyl, 2-methylcyclopropyl, cyclopropylmethyl, and the like.

Alkyl, alkenyl and alkynyl chain comprises branched and unbranched chains, substituted or non-substituted. Preferably, in the present disclosure, alkyl, alkenyl and alkynyl chain relates to unbranched and non-substituted chains.

An as example, in the present disclosure, a C₄ chain represents a branched or unbranched alkyl, alkenyl or alkynyl chain with 4 carbons.

An aspect of the present disclosure relates to a compound of formula I or formula II or a pharmaceutically acceptable salt, or ester or solvate thereof 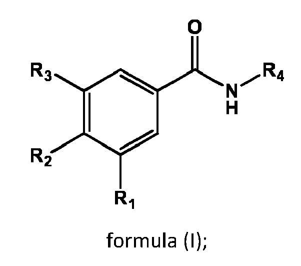 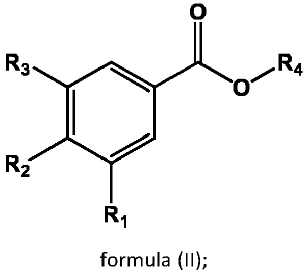
wherein R₁, R₂, R₃ and R₄ are independently selected from each other;
R₁, R₂ and R₃ are selected from N0₂, CF₃, OCF₃, F, Cl, Br, I, H, CN, OCH₃, CH₃;
R₄ is an alkyl, alkenyl or alkynyl chain with at least 3 carbons;
Provided that if R₁ and R₃ are NO₂ then R₄ is not a hexyl (C-6 alkyl) or hexadecanoyl (C16 alkyl) chain.

In an embodiment, R₄ is a C₇ chain; C₈ chain; C₉ chain; C₁₀ chain; C₁₁ chain, C₁₂ chain, C₁₃ chain, or C₁₄ chain; preferably the chain is an unsubstituted alkyl chain.

In an embodiment, R₁, R₂ and R₃ are independently select from NO₂, CF₃ and H.

In an embodiment, at least R₁ or R₂ or R₃ is NO₂.

In an embodiment, R₁ and R₃ are NO₂ and R₄ is selected from: C₄ chain; C₈ chain; C₁₀ chain; C₁₂ chain; C₁₄ chain.

In an embodiment, R₁ is CF₃ and R₃ is NO₂ and R₄ is selected from: C₄ chain; C₆ chain; C₈ chain; C₁₀ chain; C₁₂ chain.

In an embodiment, R₂ is NO₂ and R₄ is selected from: C₄ chain; C₆ chain; C₈ chain; C₁₀ chain; C₁₂ chain.

In an embodiment, the compound is:

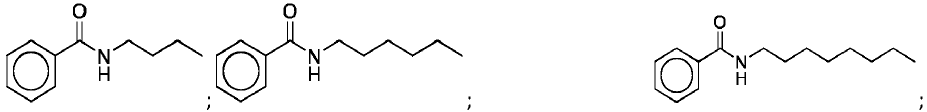

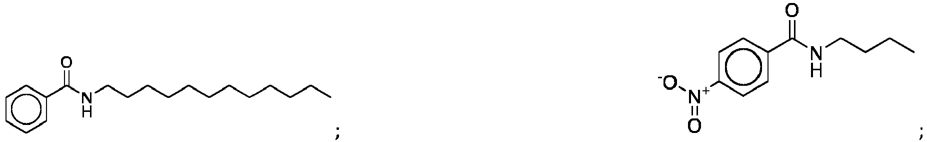

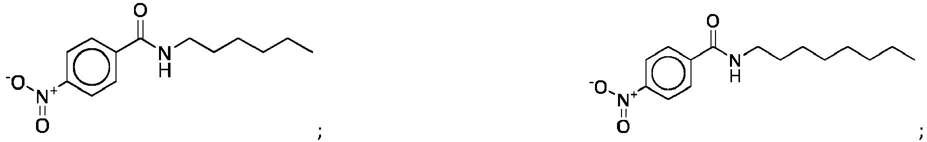

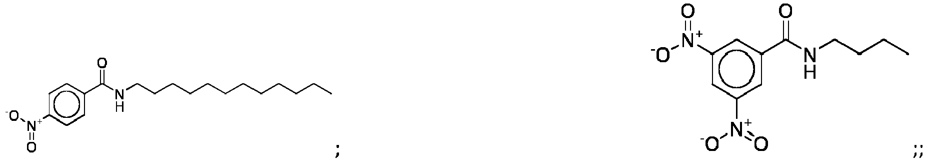

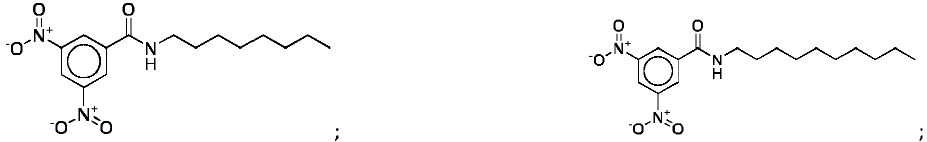

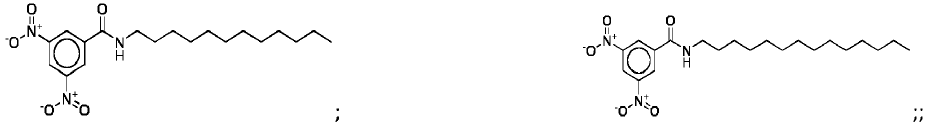

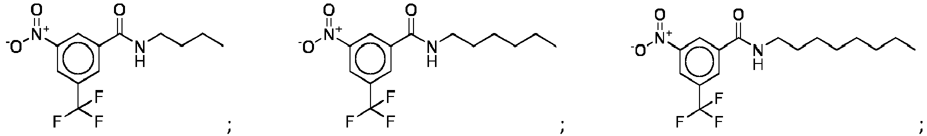

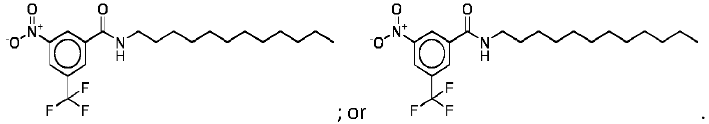

In an embodiment, the compound is:

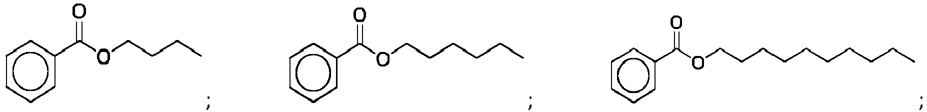

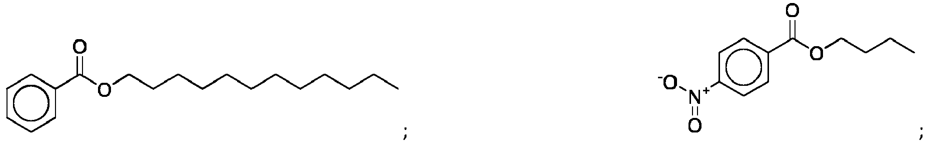

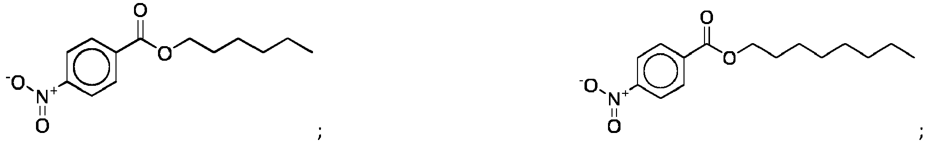

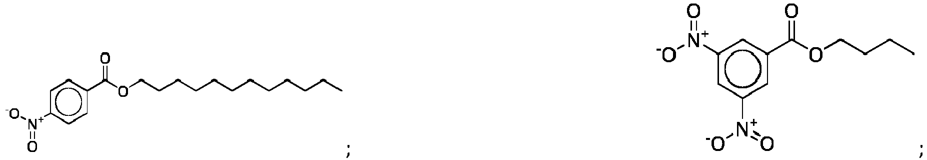

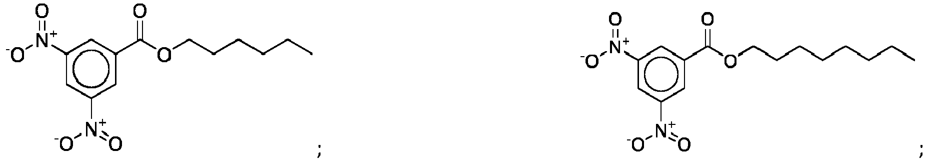

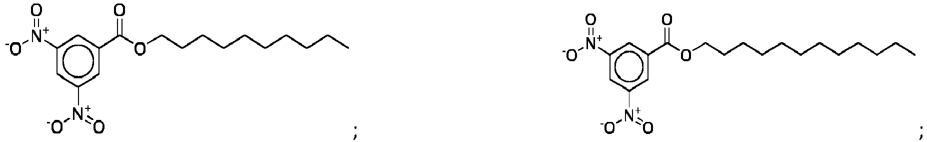

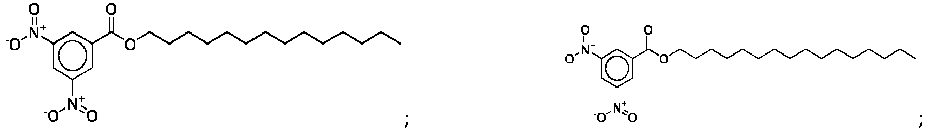

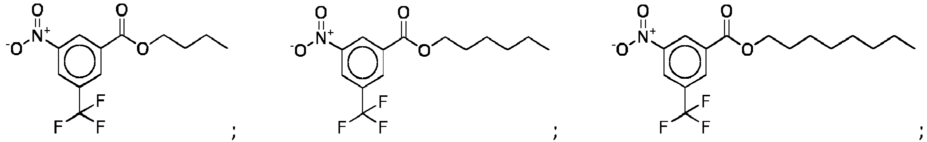

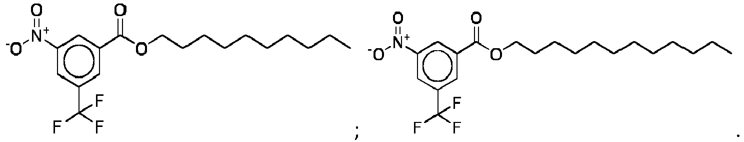

An aspect of the present disclosure relates to the use of the compounds in medicine or veterinary.

In an embodiment, the compounds may be used in any condition susceptible of being improved or prevented by selective inhibition of the enzyme DprE1.

In an embodiment, the compounds may be used in the treatment or prevention of a mycobacterial infection.

In an embodiment, the compounds may be used in the treatment or prevention of tuberculosis.

In an embodiment, tuberculosis is caused by an organism from the *Mycobacterium tuberculosis* Complex; preferably *Mycobacterium bovis* or *Mycobacterium tuberculosis.*

In an embodiment, the tuberculosis disease is selected from the group consisting of primary tuberculosis disease, post-primary pleuro-pulmonary tuberculosis disease, post-primary extra-pulmonary tuberculosis disease involving at least one organ or system of a mammal.

In an embodiment, the compounds may be used in the treatment or prevention of a mycobacterial infection, wherein the mycobacterial infection is caused by a non-tuberculous mycobacteria (NTM) selected from the group consisting of: *Mycobacterium avium* complex (MAC), *Mycobacterium smegmatis, Mycobacterium gordonae, Mycobacterium kansasii, Mycobacterium terrae, Mycobacterium scrofulaceum, Mycobacterium vaccae, Mycobacterium marinum, Mycobacterium lentiflavum, Mycobacterium fortuitum, Mycobacterium chelonae, Mycobacterium abscessus, Mycobacterium intracellulare* and *Mycobacterium avium.*

In an embodiment, the compounds may be used in combination with at least one anti-HIV agent, wherein the anti-HIV agent is selected from a HIV protease inhibitor, a HIV nucleoside reverse transcriptase inhibitor, a HIV non-nucleoside reverse transcriptase inhibitor, or a HIV integrase inhibitor.

In an embodiment, the compounds may be used in combination with at least a second tuberculosis drug, wherein the further tuberculosis drug is selected from the group of isoniazid, rifamycin and derivatives, pyrazinamide, ethambutol, cycloserine, ethionamide, streptomycin, amikacin, kanamycin, rifampin (rifampicin), aminoglycosides, capreomycin, p-aminosalicyclic acid, fluoroquinolones such as levofloxacin, moxafloxacin or gatifloxacin, or mixtures thereof.

Another aspect of the present disclosure relates to the use of the compounds as a DprE1 inhibitor.

Another aspect of the present disclosure relates to a pharmaceutical composition comprising a compound herein described and a pharmaceutical acceptable excipient.

In an embodiment, the pharmaceutical composition comprises (i) a therapeutically effective amount of a compound herein described or a pharmaceutically acceptable salt or solvate thereof; and (ii) a pharmaceutically acceptable excipient.

### DETAILED DESCRIPTION

The present disclosure relates to novel benzoic acid derivatives, specifically esters and benzamides, and their obtention. Furthermore, the present disclosure relates to the use of such compounds in medicine or veterinary, specifically, in the prevention or treatment of tuberculosis.

### Results

Twenty esters were obtained from benzoic acid (BA) or other substituted benzoic acids and divided into 4 series, as well as twenty amides derived from the same acids. The following scheme shows the general structure of the compounds synthesized

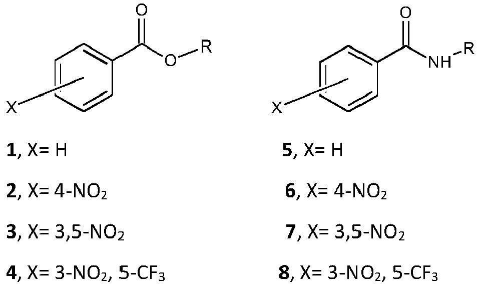

**Table 1 Chemical structure of the esters synthetized.**

| **Esters** | **X** | **R** | |
|---|---|---|---|
| **1a** | H | C₄H₉ | 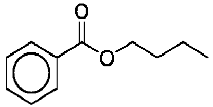 |
| **1b** | | C₆H₁₃ | 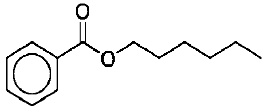 |
| **1c** | | C₈H₁₇ | 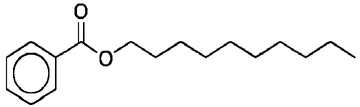 |
| 1e | | C₁₂H₂₅ | 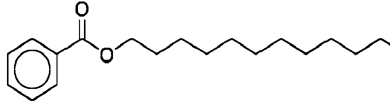 |
| **2a** | 4-NO₂ | C₄H₉ | 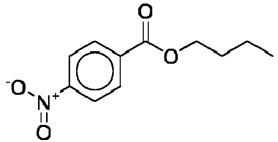 |
| **2b** | | C₆H₁₃ | 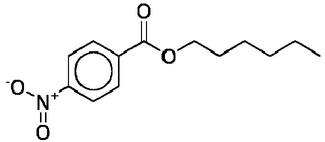 |
| **2c** | | C₈Hₗ₇ | 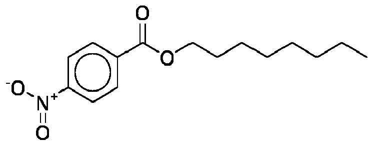 |
| 2e' | | C₁₂H₂₅ | 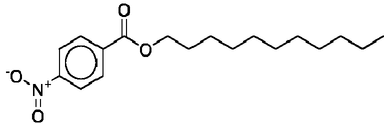 |
| **3a** | 3,5-dNO₂ | C₄H₉ | 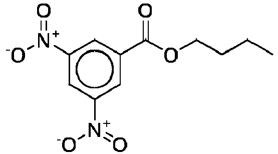 |
| **3b** | | C₆H₁₃ | 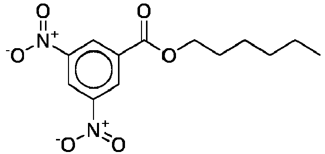 |
| **3c** | | C₈Hₗ₇ | 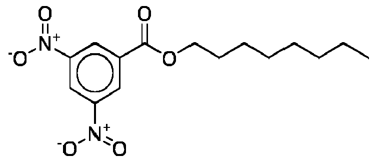 |
| **3d** | | C₁₀H₂₁ | 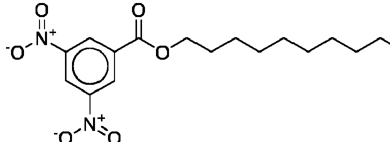 |
| **3eⁱ** | | C₁₂H₂₅ | 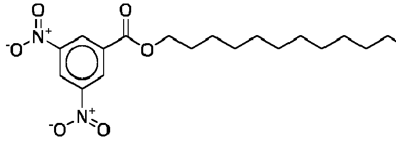 |
| **3f** | | C₁₄H₂₈ | 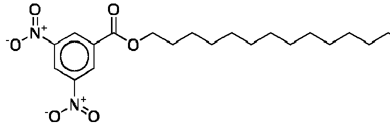 |
| **3g** | | C₁₆H₃₂ | 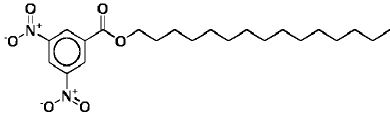 |
| **4a** | 3-NO₂-5-CF₃ | C₄H₉ | 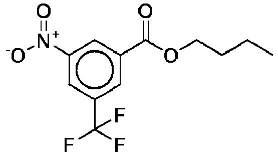 |
| **4b** | | C₆H₁₃ | 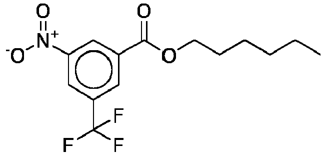 |
| **4c** | | C₈H₁₇ | 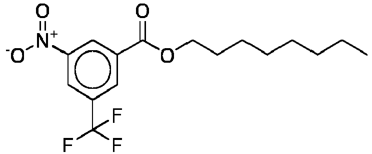 |
| **4d** | | C₁₀H₂₁ | 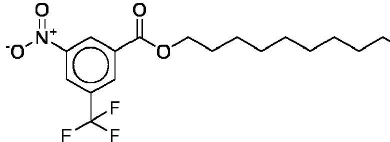 |
| **4e** | | C₁₂H₂₅ | 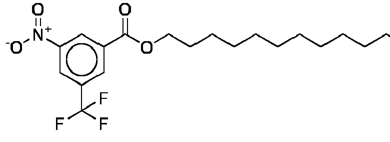 |

**Table 2 Chemical structure of the amides synthetized.**

| **Amides** | **X** | **R** | |
|---|---|---|---|
| **5a** | H | C₄H₉ | 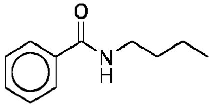 |
| **5b** | | C₆H₁₃ | 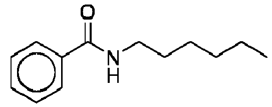 |
| **5c** | | C₈H₁₇ | 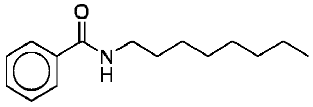 |
| **5e** | | C₁₂H₂₅ | 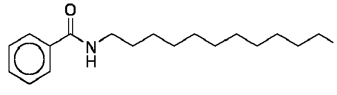 |
| **6a** | 4-NO₂ | C₄H₉ | 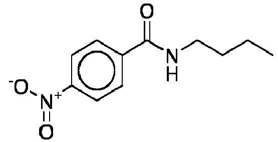 |
| **6b** | | C₆H₁₃ | 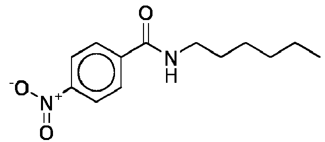 |
| **6c** | | C₈H₁₇ | 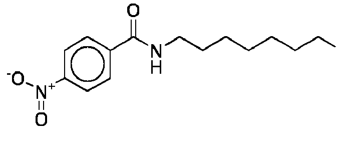 |
| **6e** | | C₁₂H₂₅ | 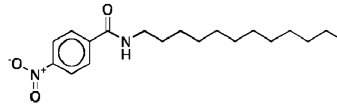 |
| **7a** | 3,5-dNO₂ | C₄H₉ | 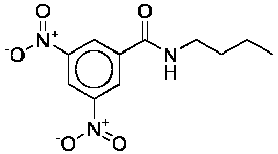 |
| **7b** | | C₆H₁₃ | 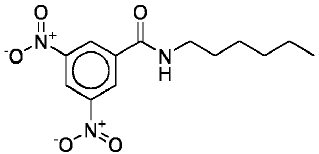 |
| **7c** | | C₈H₁₇ | 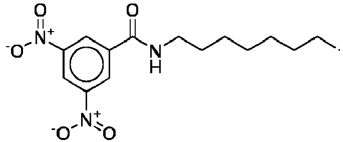 |
| **7d** | | C₁₀H₂₁ | 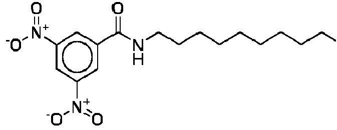 |
| **7e** | | C₁₂H₂₅ | 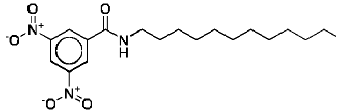 |
| 7f | | C₁₄H₂₉ | 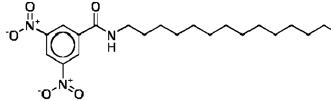 |
| **7g** | | C₁₆H₃₂ | 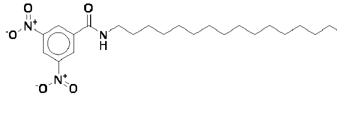 |
| **8a** | 3-NO₂-5-CF₃ | C₄H₉ | 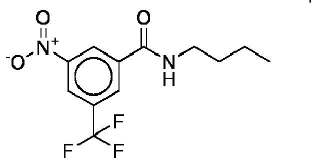 |
| **8b** | | C₆H₁₃ | 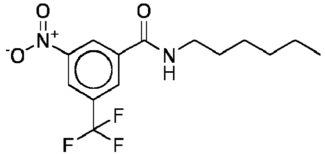 |
| **8c** | | C₈H₁₇ | 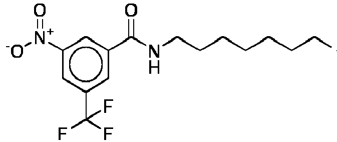 |
| **8d** | | C₁₀H₂₁ | 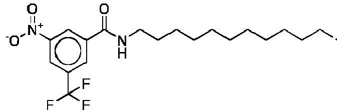 |
| **8e** | | C₁₂H₂₅ | 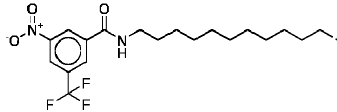 |

### Activity against M. tuberculosis

In order to evaluate whether the synthesized derivatives of the substituted benzoic acids inhibited the growth or led to the death of the mycobacteria, *in vitro* activity assays were performed using the *Mycobacterium tuberculosis* H37Rv strain. The results of the activity assays are represented as Minimum Inhibitory Concentration (MIC) and Minimum Bactericidal Concentration (MBC), in µg/mL, in **Table 3.** MIC is the lowest concentration of a compound responsible for limiting the visible growth of a bacterium. MBC corresponds to the minimum concentration of an antimicrobial agent that results in bacterial death. If the values of MIC and MBC are similar this means the compounds show bactericidal activity and if the values are MBC > MIC x3, the compound is considered bacteriostatic.

**Table 3. Structure of the compounds under study and MIC against M. tuberculosis H37Rvwith MIC and MBC values shown in (µg/mL).**

| **Esters** | **X** | **R** | **MIC** (µg/mL) | **MBC** (µg/mL) | **Amide s** | **X** | **R** | **MIC** (µg/mL) | **MBC** (µg/mL) |
|---|---|---|---|---|---|---|---|---|---|
| **1a** | H | C₄H₉ | >256 | >256 | **5a** | H | C₄H₉ | 256 | 256 |
| **1b** | | C₆H₁₃ | - | - | **5b** | | C₆H₁₃ | 64 | 64 |
| **1c** | | C₈Hₗ₇ | 256 | >256 | **5c** | | C₈Hₗ₇ | 32 | 32 |
| **1e** | | C₁₂H₂₅ | 256 | 512 | **5e** | | C₁₂H₂₅ | >256 | >256 |
| **2a** | 4-NO₂ | C₄H₉ | 64 | 128 | **6a** | 4-NO₂ | C₄H₉ | 128 | 256 |
| **2b** | | C₆H₁₃ | 64 | 128 | **6b** | | C₆H₁₃ | 32 | 64 |
| **2c** | | C₈H₁₇ | 8 | 128 | **6c** | | C₈Hₗ₇ | 128 | 256 |
| **2e** | | C₁₂H₂₅ | 8 | 64 | **6e** | | C₁₂H₂₅ | 256 | 256 |
| **3a** | 3,5-dNO₂ | C₄H₉ | 32 | 128 | **7a** | 3,5-dNO₂ | C₄H₉ | **0.5** | **0.5** |
| **3b** | | C₆H₁₃ | 32 | 4 | **7b** | | C₆H₁₃ | 0.031 | **0.031** |
| **3c** | | C₈H₁₇ | 8 | 8 | **7c** | | C₈Hₗ₇ | **0.016** | **0.016** |
| **3d** | | C₁₀H₂₁ | 4 | 4 | **7d** | | C₁₀H₂₁ | **0.063** | **0.063** |
| **3e** | | C₁₂H₂₅ | 8 | 16 | **7e** | | C₁₂H₂₅ | **0.5** | **0.5** |
| **3f** | | C₁₄H₂₉ | 16 | >1024 | **7f** | | C₁₄H₂₉ | **0.125** | **0.25** |
| **3g** | | C₁₆H₂₅ | >1024 | >1024 | **7g** | | C₁₆H₃₂ | **2** | **2** |
| **4a** | 3-NO₂-5-CF₃ | C₄H₉ | 1024 | 1024 | **8a** | 3-NO₂-5-CF₃ | C₄H₉ | **2** | **2** |
| **4b** | | C₆H₁₃ | 1024 | 1024 | **8b** | | C₆H₁₃ | **≤0.5** | **≤0.5** |
| **4c** | | C₈H₁₇ | - | - | **8c** | | C₈Hₗ₇ | **0.063** | **0.063** |
| **4d** | | C₁₀H₂₁ | 128 | 256 | **8d** | | C₁₀H₂₁ | **0.5** | **0.5** |
| **4e** | | C₁₂H₂₅ | >1024 | >1024 | **8e** | | C₁₂H₂₅ | **0.5** | **0.5** |
| **isoniazi d (INH)** | | | 0.025 | 0.05 | | | | 0.025 | 0.05 |

### Activity against other mycobacterial strains

When evaluating the results of stability (information depicted below) and activity of the tested compounds, it was found that there is no relationship between stability, activation and activity in the tested compounds. This observation is not compatible with the performance of compounds as prodrugs releasing a weak acid. The idea arose that the compounds with higher activity (3,5-dinitrobenzamides and 3-nitro-5-(trifluoromethyl)benzamides) do not require enzymatic hydrolysis and would act by another mechanism of action. An attractive explanation for their activity would be to have a mechanism of action similar to benzothiazinones (BTZ) that have a nitro group and are already known in antimycobacterial therapy. BTZ compounds act on decaprenilphosphoryl-β-d-ribose 2'-epimerase (DprE1).

An experiment was carried out in which the activity of the compounds on four species of mycobacteria was tested: *M*. *avium, M. smegmatis, M. bovis* BCG and *M*. *tuberculosis.* These results are depicted in **Table 3.** *M*. *avium* DSM species does not have the Cys387 residue, since the gene encoding for the enzyme DprE1 has the cysteine replaced by alanine.[8]. These mutations confer natural resistance to the tested compounds. *M. smegmatis* mc2 has overexpression of NfnB nitroresductase leading to potential inactivation of the drug by reducing one nitro group to an amino group [9]. The other two species (*M*. *bovis* BCG, *M*. *tuberculosis* H37Rv) are susceptible to DprE1 inhibitors. When evaluating the values obtained in Table 3, it was found that tested amides showed high activity in *M*. *bovis* and *M*. *tuberculosis,* however, the activity is completely lost in *M*. *avium,* which has mutation in the enzyme DprE1.

Similar results were presented by benzothiazine BTZ047, which was tested by Caroline et al, [8] where BTZ047 showed high activity in *M*. *tuberculosis,* however, the activity was lost in *M*. *avium* where cys387 residue is replaced by alanine [8]. Therefore, the residue Cys387 of DprE1 is necessary for covalent interaction with nitroaromatic inhibitors and substitutions in this residue confer resistance to the compounds tested.

Although the activity in *M. smegmatis* is not completely lost, it is decreased which may mean that some amount of compound suffered the nitro group reduction before entering the active site of DprE1. In particular, it has been shown that the overexpression of FMN-dependent NfnB nitroreductase in *M. smegmatis* may be a new mechanism for partial resistance of tested compounds involving inactivation of compounds, in addition to the one already marked above amino acid substitution for Cys387 DprE1. The set of these results indicates that the target for the most active compounds may be the enzyme DprE1. Of course, further studies outside the scope of this thesis will be necessary to prove the target of the compounds.

**Table 4. Activity of the compounds on four species of mycobacteria.**

| **Compound** | **R** | ***M. avium* DSM 44156** | | ***M. smegmatis* mc2 155** | | ***M. bovis* BCG** | | ***M. tuberculosis* H37Rv** | |
|---|---|---|---|---|---|---|---|---|---|
| | | **MIC [µg/mL]** | **MBC [µg/mL]** | **MIC [µg/mL]** | **MBC [µg/mL]** | **MIC [µg/mL]** | **MBC [µg/mL]** | **MIC [µg/mL]** | **MBC [µg/ mL]** |
| **7a** | C₄H₉ | 64 | 512 | 4 | 4 | 0.5 | 1 | 0.5 | 0.5 |
| **7b** | C₆H₁₃ | ND | ND | 1 | 1 | 0.25 | 0.25 | ≤0.5 | ≤0.5 |
| **7c** | C₈H₁₇ | 32 | 64 | 0.25 | 4 | 0.015 | 0.015 | ≤0.062 | ≤0.062 |
| **7d** | C₁₀H₂₁ | ND | ND | 1 | 1 | 0.062 | 0.062 | ≤0.5 | ≤0.5 |
| **7e** | C₁₂H₂₅ | >341 | >341 | 0.66 | >2.66 | 0.083 | 0.166 | 0.5 | 0.5 |
| **7f** | C₁₄H₂₉ | ND | ND | 1 | 4 | 0.25 | 0.25 | ≤0.5 | ≤0.5 |
| **7g** | C₁₆H₃₃ | ND | ND | 32 | >64 | 2 | 4 | 2 | 2 |

### Activity tests in macrophages

To evaluate the ability of the compounds under study to act within the host cell, activity was evaluated in human THP-1 macrophages infected by *Mycobacterium bovis* BCG. Three esters and three amides derived from 3.5-dinitrobenzoic acid were accessed. As controls, DMSO and isoniazid (INH) were used. The results obtained are depicted in **Table 5.**

**Table 5. Intracellular antimicrobial activity of compounds tested against Mycobacterium Bovis BCG infecting human macrophages THP-1. The results are presented in CFUs per mL.**

| **CFU x10^4** | | **Compound (concentration 2 µg/ml):** | | | | | | 0.**1 µg/ml** |
|---|---|---|---|---|---|---|---|---|
| Time (days) | **DMSO** | 7b | 7d | 7f | 3b | 3d | 3f | **INH** |
| 0 | 9.2 | 9.2 | 9.2 | 9.2 | 9.2 | 9.2 | 9.2 | 9.2 |
| 1 | 15.33 | 4.58 | 7.33 | 14.16 | 14.58 | 12.5 | 12.16 | 9.91 |
| 3 | 10.75 | 0.40 | 0.32 | 10.08 | 11.87 | 12.5 | 13.66 | 1.33 |
| 7 | 11.91 | 0.05 | 0.03 | 13.91 | 9.83 | 8.5 | 7.25 | 0.24 |

### Cytotoxicity assays

The metabolization of nitro compounds can lead to the formation of toxic, genotoxic, mutagenic and/or carcinogenic intermediates and this is undoubtedly the reason for avoiding these compounds in most cases. Many nitro compounds can generate reactive oxygen and nitrogen species that can react with biomolecules. In this way, these compounds have attracted considerable attention due to their potential risk to human health.

To study whether nitro esters/amides of BA derivatives showed toxicity, the cytotoxicity of these human monocytic cell line esters THP-1 (ATCC56 TIB202) was studied. The results are depicted in **Table 6** and **Table 7.** Cytotoxicity results were obtained through lethal concentration for 50% of the cell population, LC₅₀.

**Table 6. Results obtained from cytotoxicity assays for the esters, represented by LD₅₀ in µg/mL and comparison with MIC values, in µg/mL with toxicity/activity (T/A) index.**

| **Esters** | **X** | **R** | **MIC** (µg/mL) | **LC₅₀** (µg/mL) | T/A |
|---|---|---|---|---|---|
| **1c** | H | C₈H₁₇ | 256 | 1646 | 6 |
| **2c** | 4-NO₂ | C₈H₁₇ | 8 | 657 | 82 |
| **3a** | 3,5-dNO₂ | C₄H₉ | 32 | 456 | 14 |
| **3b** | | C₆H₁₃ | 32 | 528 | 17 |
| **3c** | | C₈H₁₇ | 8 | n//d | - |
| **3d** | | C₁₀H₂₁ | 4 | n/d | - |
| **3e** | | C₁₂H₂₅ | 8 | n/d | - |
| **3f** | | C₁₄H₂₉ | 16 | n/d | - |
| **3g** | | C₁₆H₂₅ | n/d | n/d | - |
| **4a** | 3-NO₂-5-CF₃ | C₄H₉ | 1024 | 552 | 0.54 |
| **4b** | | C₆H₁₃ | 1024 | 639 | 0.62 |
| **4c** | | C₈H₁₇ | - | - | |
| **4d** | | C₁₀H₂₁ | 128 | n/d | - |
| **4e** | | C₁₂H₂₅ | >1024 | n/d | - |

| | | | | | |
|---|---|---|---|---|---|
| n/d - Do not present any toxicity at maximum doses that have been tested (512 µg/ml). | | | | | |

**Table 7. Results obtained from cytotoxicity assays for the amides, represented by LD₅₀ in µg/mL and comparison with MIC values, in µg/mL with toxicity/activity (T/A) index**

| **Amides** | **X** | **R** | **MIC** (µg/mL) | **LC₅₀** (µg/mL) | T/A |
|---|---|---|---|---|---|
| **5c** | H | C₈H₁₇ | 32 | 188 | 6 |
| **6c** | 4-NO₂ | C₈H₁₇ | 128 | 308 | 2 |
| **7a** | 3,5-dNO₂ | C₄H₉ | 0.5 | 26 | 52 |
| **7b** | | C₆H₁₃ | 0.031 | 361 | 11645 |
| **7c** | | C₈Hₗ₇ | 0.016 | 97 | 6063 |
| **7d** | | C₁₀H₂₁ | 0.063 | 53 | 841 |
| **7e** | | C₁₂H₂₅ | 0.5 | 72 | 144 |
| **7f** | | C₁₄H₂₉ | 0.125 | 330 | 2640 |
| **7g** | | C₁₆H₃₂ | 2 | n/d | - |
| **8a** | 3-NO₂-5-CF₃ | C₄H₉ | 2 | 41 | 21 |
| **8b** | | C₆H₁₃ | ≤0.5 | 23 | 46 |
| **8c** | | C₈H₁₇ | 0.063 | 14 | 222 |
| **8d** | | C₁₀H₂₁ | 0.5 | 53 | 106 |
| **8e** | | C₁₂H₂₅ | 0.5 | 113 | 226 |

The T/A index quantitatively is the ratio between the lethal concentration (LC₅₀) given by the minimum inhibitory concentration (MIC). A low T/A indicates that potentially compounds that can reach toxic levels with ease.

It was verified that the esters tested did not present toxicity at the maximum doses that were tested (512 µg/mL) with the exception of one compound (3a) that presented LC₅₀ equal to 456 µg/mL thus forming the T/A index≈14.

The amides in turn present LC₅₀ between 14 µg/mL and 360 µg/mL. However, it was surprisingly found that the LC₅₀ of the amides with activity (3.5-dinitrobenzamides and 3-nitro-5(trifluoromethyl)benzamides) is much higher than the MIC, resulting in high levels of the toxicity/activity index. For example, compound 7b has a T/A=11645 index, which means that the LC₅₀ of that compound is 11645 times greater than the MIC value of the compound.

These results showed that contrary to what might be expected, bearing in mind that nitro group is often associated with toxicity phenomena, despite being toxic to mycobacteria, the nitro compounds have little effect on the survival of macrophages and have not shown high toxicity, especially in the most active compounds, which is an important characteristic for the compounds developed.

### Stability Studies of Esters and Amides of Benzoic Acid Derivatives

The synthesized compounds were studied for their chemical and enzymatic stability. Kinetic studies were conducted in phosphate buffer at pH 7.4 (to evaluate the chemical stability), in human plasma (to evaluate plasma stability) and in *Mycobacterium smegmatis* homogenate (to evaluate the potential reaction of enzymatic activation in mycobacteria).

### Stability in phosphate buffer pH 7.4 .

Chemical hydrolysis was evaluated in phosphate buffer at physiological pH (7.4) to determine its influence in enzymatic studies. From the determination of acid concentrations, the percentages of ester/amide degradation were calculated as shown in **Table 8** and **Table 9.**

**Table 8. Results obtained from the percentage values of ester degradation after 14 days in phosphate buffer pH 7.4.**

| **Esters** | **X** | **R** | **Degradation (%)** | | | | |
|---|---|---|---|---|---|---|---|
| | | | **24h** | **48h** | **72h** | **168h** | **332h** |
| **1a** | H | C₄H₉ | 0 | 0 | 0 | 1 | 2 |
| **1c** | | C₈Hₗ₇ | 0 | 0 | 0 | 0 | 0 |
| **1e** | | C₁₂H₂₅ | 0 | 0 | 0 | 0 | 0 |
| **2a** | 4-NO₂ | C₄H₉ | 0 | 1 | 1 | 2 | 4 |
| **2c** | | C₈Hₗ₇ | 0 | 1 | 1 | 3 | 6 |
| **2e** | | C₁₂H₂₅ | 0 | 0 | 0 | 1 | 1 |
| **3a** | 3,5-dNO₂ | C₄H₉ | 10 | 14 | 18 | 40 | 65 |
| **3c** | | C₈H₁₇ | 0 | 0 | 1 | 2 | 3 |
| **3e** | | C₁₂H₂₅ | 0 | 0 | 1 | 1 | 2 |
| **4a** | 3-NO₂-5-CF₃ | C₄H₉ | 5 | 12 | 16 | 35 | 60 |
| **4c** | | C₈H₁₇ | 0 | 1 | 1 | 3 | 6 |
| **4e** | | C₁₂H₂₅ | 1 | 2 | 3 | 6 | 11 |

**Table 9. Results obtained from the percentage values of amide degradation after 14 days in phosphate buffer pH 7.4.**

| **Amides** | **X** | **R** | **Degradation (%)** | | | | |
|---|---|---|---|---|---|---|---|
| | | | **24h** | **48h** | **72h** | **168h** | **332h** |
| **5a** | H | C₄H₉ | 0 | 0 | 0 | 0 | 0 |
| **5c** | | C₈H₁₇ | 0 | 0 | 0 | 0 | 0 |
| **5e** | | C₁₂H₂₅ | 0 | 0 | 0 | 0 | 0 |
| **6a** | 4-NO₂ | C₄H₉ | 0 | 0 | 0 | 0 | 0 |
| **6c** | | C₈H₁₇ | 0 | 0 | 0 | 0 | 0 |
| **6e** | | C₁₂H₂₅ | 0 | 0 | 0 | 0 | 0 |
| **7a** | 3,5-dNO₂ | C₄H₉ | 0 | 0 | 0 | 0 | 0 |
| **7c** | | C₈H₁₇ | 0 | 0 | 0 | 0 | 0 |
| **7e** | | C₁₂H₂₅ | 0 | 0 | 0 | 0 | 0 |
| **8a** | 3-NO₂-5-CF₃ | C₄H₉ | 0 | 0 | 0 | 0 | 0 |
| **8c** | | C₈H₁₇ | 0 | 0 | 0 | 0 | 0 |
| **8e** | | C₁₂H₂₅ | 0 | 0 | 0 | 0 | 0 |

It was observed that Most of the compounds were very stable in PBS at pH7.4. Still esters present some degradation while amides, in turn, do not present relevant degradation during the study **(Table 9).**

### Stability in human plasma

The studies of stability in human plasma were performed in human plasma diluted to 80% with phosphate buffer pH 7.4 of total concentration 0.05 M and ionic strength 0.15 M at 37°C. The results are depicted in **Table 10.**

**Table 10. Study of enzymatic hydrolysis. Reaction rate constants and half-life times of esters of benzoic acid derivatives replaced in human plasma at 37°C temperature for 72h. Initial substrate concentration (ester) - 5×10-4M with 2% ACN.**

| **Esters** | **X** | **R** | *k*_{obs} × *100* (h⁻¹) | 1/2 (h) |
|---|---|---|---|---|
| **1a** | H | C₄H₉ | 44.1 ± 6.1 | 1.59 ± 0.21 |
| **1b** | | C₆H₁₃ | 11.6 ± 1.2 | 6.00 ± 0.61 |
| **1c** | | C₈H₁₇ | 2.66 ± 0.23 | 26.1 ± 2.4 |
| **1e** | | C₁₂H₂₅ | n/d | n/d |
| **2a** | 4-NO₂ | C₄H₉ | 132 ± 6 | 0.53 ± 0.01 |
| **2b** | | C₆H₁₃ | 27.4 ± 5.8 | 2.62 ± 0.63 |
| **2c** | | C₈Hₗ₇ | 9.73 ± 1.20 | 7.18 ± 0.9 |
| **2eⁱ** | | C₁₂H₂₅ | 0.66 ± 0.10 | 106 ± 17 |
| **3a** | 3,5-dNO₂ | C₄H₉ | 8.08 ± 0.29 | 8.59 ± 0.31 |
| **3b** | | C₆H₁₃ | 5.68 ± 0.47 | 12.3 ±1.0 |
| **3c** | | C₈H₁₇ | 3.53 ± 0.44 | 19.9 ± 2.6 |
| **3d** | | C₁₀H₂₁ | 0.98 ± 0.07 | 71.0 ± 4.7 |
| **3eⁱ** | | C₁₂H₂₅ | 0.89 ± 0.13 | 78.4 ± 11.7 |
| **3f** | | C₁₄H₂₈ | 0.97 ± 0.23 | 74.1 ± 18.7 |
| **3g** | | C₁₆H₃₂ | n/d | n/d |
| **4a** | 3-NO₂-5-CF₃ | C₄H₉ | 12.5 ± 1.8 | 5.63 ± 0.88 |
| **4b** | | C₆H₁₃ | 5.96 ± 1.28 | 12.0 ± 2.4 |
| **4c** | | C₈H₁₇ | 2.30 ± 0.06 | 30.1 ± 0.9 |
| **4d** | | C₁₀H₂₁ | 1.26 ± 0.04 | 55.0 ± 1.7 |
| **4e** | | C₁₂H₂₅ | n/d | n/d |

The amides showed virtually no degradation during 72h and only one amide, derived from 4-nitrobenzoic acid (6a) showed 32% degradation over 72h. Since the degradation rate was very low, it was not possible to calculate the rate constants (kobs) or the half-life times (t1/2). Hence it was decided to compare the percentage of degradation after 24h, 48h and 72h of esters and amides **(Table 11 and Table 12).**

**Table 11. Results obtained from the percentage values of ester degradation after 72 hours in an enzymatic stability assay: human plasma.**

| **Esters** | **X** | **R** | **Degradation (%)** | | |
|---|---|---|---|---|---|
| | | | **24h** | **48h** | **72h** |
| **1a** | H | C₄H₉ | 100 | 100 | 100 |
| **1b** | | C₆H₁₃ | 94 | 100 | 100 |
| **1c** | | C₈H₁₇ | 45 | 71 | 84 |
| **1e** | | C₁₂H₂₅ | 3 | 6 | 9 |
| **2a** | 4-NO₂ | C₄H₉ | 100 | 100 | 100 |
| **2b** | | C₆H₁₃ | 100 | 100 | 100 |
| **2c** | | C₈H₁₇ | 100 | 100 | 100 |
| **2e** | | C₁₂H₂₅ | 15 | 27 | 38 |
| **3a** | 3,5-dNO₂ | C₄H₉ | 86 | 98 | 100 |
| **3b** | | C₆H₁₃ | 74 | 93 | 98 |
| **3c** | | C₈H₁₇ | 57 | 81 | 92 |
| **3d** | | C₁₀H₂₁ | 18 | 32 | 44 |
| **3e** | | C₁₂H₂₅ | 29 | 50 | 65 |
| **3f** | | C₁₄H₂₈ | 23 | 51 | 65 |
| **3g** | | C₁₆H₃₂ | 1 | 3 | 4 |
| **4a** | 3-NO₂-5-CF₃ | C₄H₉ | 95 | 100 | 100 |
| **4b** | | C₆H₁₃ | 72 | 94 | 98 |
| **4c** | | C₈H₁₇ | 42 | 67 | 81 |
| **4d** | | C₁₀H₂₁ | 26 | 45 | 60 |
| **4e** | | C₁₂H₂₅ | 1 | 2 | 3 |

**Table 12. Results obtained from the percentage values of amide degradation after 72 hours in an enzymatic stability assay: human plasma.**

| **Amides** | **X** | **R** | **Degradation (%)** | | |
|---|---|---|---|---|---|
| | | | **24h** | **48h** | **72h** |
| **5a** | H | C₄H₉ | 0 | 0 | 0 |
| **5b** | | - | - | - | - |
| **5c** | | C₈H₁₇ | 0 | 0 | 0 |
| **5e** | | C₁₂H₂₅ | 0 | 0 | 0 |
| **6a** | 4-NO₂ | C₄H₉ | 12 | 23 | 32 |
| | | - | - | - | - |
| **6c** | | C₈H₁₇ | 0 | 0 | 1 |
| **6e** | | C₁₂H₂₅ | 0 | 0 | 1 |
| **7a** | 3,5-dNO₂ | C₄H₉ | 0 | 0 | 0 |
| | | | | | |
| **7c** | | C₈H₁₇ | 0 | 0 | 0 |
| | | | | | |
| **7e** | | C₁₂H₂₅ | 0 | 0 | 0 |
| | | | | | |
| | | | | | |
| **8a** | 3-NO₂-5-CF₃ | C₄H₉ | 0 | 0 | 0 |
| | | | | | |
| **8c** | | C₈H₁₇ | 0 | 0 | 0 |
| | | | | | |
| **8e** | | C₁₂H₂₅ | 0 | 0 | 0 |

### Stability in Mycobacterium smegmatis homogenate

These studies were carried out in homogenate solutions of mycobacteria diluted at 2% in phosphate buffer pH = 7.4 of total concentration 0.05M and ionic strength at 0.15M at 37°C. The values of pseudo-first order (kobs) constants obtained for the activation of prodrugs by M. *smegmatis* homogenate are represented in **Table 13 and Table 14,** and 3 trials were performed for each compound.

It was found, as in the other biological media studied previously, that amides are much more resistant to hydrolysis than the corresponding esters. Since the degradation rate has a very low value, the rate constants (kobs) and the half-life times (t_{1/2}) were calculated only for short chains, which present the highest level of degradation. For long chains, only the percentages of amide degradation are displayed **(Table 15 and Table 16).**

**Table 13 Study of enzymatic hydrolysis. Reaction rate constants and half-life times of esters in Mycobacterium smegmatis homogenate at 37°C temperature for 48h. Initial substrate concentration - 5×10⁻⁴M with 2% ACN.**

| **Este rs** | **X** | **R** | *k*_{obs} × *100* (h⁻¹) | t_{1/2} (h) |
|---|---|---|---|---|
| **1a** | H | C₄H₉ | 356 ± 17 | 0.20 ± 0.01 |
| **1b** | | C₆H₁₃ | 332 ± 19 | 0.21 ± 0.01 |
| **1c** | | C₈H₁₇ | 35.5 ± 0.3 | 1.96 ± 0.02 |
| **1e** | | C₁₂H₂₅ | 0.76 ± 0.02 | 91.4 ± 3.1 |
| **2a** | 4-NO₂ | C₄H₉ | 20.4 ± 3.3 | 3.46 ± 0.49 |
| **2b** | | C₆H₁₃ | 6.05 ± 0.31 | 11.5 ± 0.6 |
| **2c** | | C₈H₁₇ | 1.80 ± 0.38 | 39.0 ± 7.7 |
| **2e** | | C₁₂H₂₅ | 0.88 ± 0.05 | 79.4 ± 4.7 |
| **3a** | 3,5-dNO₂ | C₄H₉ | 11.0 ± 0.8 | 6.32 ± 0.44 |
| **3b** | | C₆H₁₃ | 0.86 ± 0.06 | 80.9 ± 5.5 |
| **3c** | | C₈H₁₇ | n/d | n/d |
| **3d** | | C₁₀H₂₁ | n/d | n/d |
| **3e** | | C₁₂H₂₅ | n/d | n/d |
| **3f** | | C₁₄H₂₉ | n/d | n/d |
| **3g** | | C₁₆H₂₅ | n/d | n/d |
| **4a** | 3-NO₂-5-CF₃ | C₄H₉ | 13.0 ± 1.1 | 5.36 ± 0.44 |
| **4b** | | C₆H₁₃ | 0.67 ± 0.06 | 105 ± 10 |
| **4c** | | C₈H₁₇ | n/d | n/d |
| **4d** | | C₁₀H₂₁ | n/d | n/d |
| **4e** | | C₁₂H₂₅ | n/d | n/d |

**Table 14 Study of enzymatic hydrolysis. Reaction rate constants and half-life times of amides in Mycobacterium smegmatis homogenate at 37°C temperature for 48h. Initial substrate concentration - 5×10⁻⁴M with 2% ACN.**

| **Amides** | **X** | **R** | *k*_{obs} × *100* (h⁻¹) | t_{1/2} (h) |
|---|---|---|---|---|
| **5a** | H | C₄H₉ | n/d | n/d |
| **5b** | | C₆H₁₃ | n/d | n/d |
| **5c** | | C₈Hₗ₇ | n/d | n/d |
| **5e** | | C₁₂H₂₅ | n/d | n/d |
| **6a** | 4-NO₂ | C₄H₉ | 0.91 ± 0.07 | 76.5 ± 5.3 |
| **6b** | | C₆H₁₃ | 0.80 ± 0.05 | 87.2 ± 5.8 |
| **6c** | | C₈H₁₇ | n/d | n/d |
| **6e** | | C₁₂H₂₅ | n/d | n/d |
| **7a** | 3,5-dNO₂ | C₄H₉ | 3.03 ± 0.20 | 22.9 ± 1.5 |
| **7b** | | C₆H₁₃ | 0.84 ± 0.01 | 82.5 ± 1.4 |
| **7c** | | C₈H₁₇ | n/d | n/d |
| **7d** | | C₁₀H₂₁ | n/d | n/d |
| **7e** | | C₁₂H₂₅ | n/d | n/d |
| **7f** | | C₁₄H₂₉ | n/d | n/d |
| **7g** | | C₁₆H₃₂ | n/d | n/d |
| **8a** | 3-NO₂-5-CF₃ | C₄H₉ | 1.09 ± 0.07 | 63.8 ± 4.1 |
| **8b** | | C₆H₁₃ | n/d | n/d |
| **8c** | | C₈H₁₇ | n/d | n/d |
| **8d** | | C₁₀H₂₁ | n/d | n/d |
| **8e** | | C₁₂H₂₅ | n/d | n/d |

**Table 15 Study of enzymatic hydrolysis. Percentage of degradation of esters in Mycobacterium smegmatis homogenate at 37°C temperature for 48h. Initial substrate concentration - 5×10⁻⁴M with 2% ACN.**

| **Esters** | **X** | **R** | **Degradation (%)** | |
|---|---|---|---|---|
| | | | **24h** | **48h** |
| **1a** | H | C₄H₉ | 100 | 100 |
| **1b** | | C₆H₁₃ | 100 | 100 |
| **1c** | | C₈H₁₇ | 100 | 100 |
| **1e** | | C₁₂H₂₅ | 16 | 30 |
| **2a** | 4-NO₂ | C₄H₉ | 99 | 100 |
| **2b** | | C₆H₁₃ | 77 | 94 |
| **2c** | | C₈H₁₇ | 35 | 58 |
| **2e** | | C₁₂H₂₅ | 19 | 34 |
| **3a** | 3,5-dNO₂ | C₄H₉ | 93 | 99 |
| **3b** | | C₆H₁₃ | 19 | 34 |
| **3c** | | C₈H₁₇ | 10 | 19 |
| **3d** | | C₁₀H₂₁ | 5 | 9 |
| **3e** | | C₁₂H₂₅ | 4 | 8 |
| **3f** | | C₁₄H₂₉ | 3 | 6 |
| **3g** | | C₁₆H₂₅ | 2 | 4 |
| **4a** | 3-NO₂-5-CF₃ | C₄H₉ | 95 | 100 |
| **4b** | | C₆H₁₃ | 15 | 27 |
| **4c** | | C₈H₁₇ | 8 | 16 |
| **4d** | | C₁₀H₂₁ | 5 | 10 |
| **4e** | | C₁₂H₂₅ | 3 | 6 |

**Table 16. Study of enzymatic hydrolysis. Percentage of degradation of amides in Mycobacterium smegmatis homogenate at 37°C temperature for 48h. Initial substrate concentration - 5×10⁻⁴M with 2% ACN.**

| **Amides** | **X** | **R** | **Degradation (%)** | |
|---|---|---|---|---|
| | | | **24h** | **48h** |
| **5a** | H | C₄H₉ | 2 | 3 |
| **5b** | | C₆H₁₃ | 1 | 2 |
| **5c** | | C₈Hₗ₇ | 1 | 2 |
| **5e** | | C₁₂H₂₅ | 0 | 0 |
| **6a** | 4-NO₂ | C₄H₉ | 20 | 35 |
| **6b** | | C₆H₁₃ | 17 | 32 |
| **6c** | | C₈Hₗ₇ | 3 | 5 |
| **6e** | | C₁₂H₂₅ | 2 | 3 |
| **7a** | 3,5-dNO₂ | C₄H₉ | 52 | 77 |
| **7b** | | C₆H₁₃ | 18 | 33 |
| **7c** | | C₈Hₗ₇ | 3 | 5 |
| **7d** | | C₁₀H₂₁ | 1 | 2 |
| **7e** | | C₁₂H₂₅ | 1 | 2 |
| **7f** | | C₁₄H₂₉ | 1 | 2 |
| **7g** | | C₁₆H₃₂ | 0 | 0 |
| **8a** | 3-NO₂-5-CF₃ | C₄H₉ | 23 | 41 |
| **8b** | | C₆H₁₃ | 7 | 14 |
| **8c** | | C₈Hₗ₇ | 5 | 11 |
| **8d** | | C₁₀H₂₁ | 2 | 3 |
| **8e** | | C₁₂H₂₅ | 1 | 3 |

Since one of the classes of compounds that are here presented are esters, the chemical, plasmatic, and mycobacterial homogenate stability were determined, to ensure that the compounds are stable in human plasma in order to reach the target without being degraded. The stability in buffer serves to verify whether hydrolysis in plasma can be explained by purely chemical hydrolysis or whether it corresponds to (faster) enzymatic hydrolysis.

In these assays, it was observed that compounds with longer alkyl chain lengths would be more stable and therefore more difficult to hydrolyse than those with shorter alkyl chains. The nitrated compounds proved to be chemically stable over the time interval required to perform the activity and stability tests, and the enzymatic hydrolysis was not significantly influenced by the chemical hydrolysis of the esters.

Regarding enzymatic stability, the esters show adequate stability in human plasma, however, in stability in mycobacteria the percentage degradation value was low. Thus, they should reach the target without being degraded, so they are not significantly transformed into their free acid. The amides show high stability in both human plasma and mycobacteria, so they are not transformed.

In addition to stability, the activity of these compounds against M. *tuberculosis* H37Rv was also evaluated using the MIC and MBC. It was concluded that the 3,5-dinitrobenzoate esters were the most active than the 4-nitrobenzoate esters. It was observed that for antimycobacterial activity the position of a nitro group in the aromatic moiety is important. Unexpectedly 3,5-dinitrobenzamides and 3-nitro-5-(trifluoromethyl)benzamides showed greatly improved activity compared to the respective esters. Furthermore, when comparing 3,5-dinitrobenzamides and 3-nitro-5-(trifluoromethyl)benzamides activity it was found that both groups of compounds have similar activity which allowed to conclude that only one nitro group in the aromatic zone is important for antimycobacterial activity.

It was observed that these compounds are acting as drugs.

To evaluate their ability to act within the host cell, activity on THP-1 human macrophages infected with *Mycobacterium bovis* BCG was evaluated. It was shown that the compounds can also penetrate macrophages and retain their bactericidal activity.

Since it was found that the compounds showing the highest activity also showed high enzymatic stability, the idea arose that these compounds do not require activation by mycobacterial hydrolases. Hence it is possible that the compounds of the present disclosure act in a similar way as the benzothiazinones, compounds already known to treat tuberculosis and that have structural similarity with our active compounds, and that target the enzyme DprE1. These compounds can interact via their nitro group with a cysteine residue of decaprenyl-β-d-ribose 2'-epimerase (DprE1), also covalently or otherwise, thereby blocking the first step in the epimerization reaction from decaprenyl-phosphoryl-d-ribose (DPR) to decaprenyl-phosphoryl-d-arabinose (DPA), the donor of arabinan to arabinogalactan.

Finally, cytotoxicity assays were performed, in which the LC₅₀ value and toxicity/activity index were obtained for each synthesized compound. These results showed that contrary to what might be expected, despite being toxic to mycobacteria, the compounds with nitro groups had the ability to maintain macrophage survival.

### Pharmaceutical formulations containing the compounds of the present invention

To obtain pharmaceutical formulations of the compounds amenable to being administered enterally or parenterally, liposomes containing compounds 7c and 7d were prepared according to the method described later. Briefly, Dimyristoylphosphatidylcholine (DMPC), dimyristoylphosphatidylglycerol (DMPG) and the test compound were weighed to be in the desired molar ratio, transferred to a round bottom flask, dissolved in dichloromethane, and the organic solvent was evaporated in an evaporator to form a lipid film. 1 ml of isotonic phosphate buffer pH 7.4 (PBS) was added to the lipid film and the flask was stirred to hydrate the film completely, after which the mixture was placed in an ultrasonic bath for 3 periods of 2 minutes, interspersed with 2 minutes resting time.

The following tables show encapsulation efficiency (EE) results obtained with various formulations of different compounds.

| **Compound** | **Lipids** | **EE** |
|---|---|---|
| 7c | DMPC:DMPG 7:3 | 93% |
| 7d | DMPC:DMPG 9:1 | 96% |

All formulations obtained showed high EE. The formulations if obtained under sterile conditions can be administered enterically. Although unusual the liposomal formulations can also be used to solubilize the drug to obtain an oral formulation or one that can be administered in aerosol form. One skilled in the art can easily obtain other pharmaceutical formulations with the compounds of the present invention.

### Materials and Methods

### Materials

Balanced salt solution, phosphate-buffered saline (PBS), Dulbecco's modified Eagle's medium (DMEM), and L-glutamine were purchased from Invitrogen. Sodium dodecyl sulfate (SDS), Triton X-100, benzoic acid, 4-chlorobenzoic acid, 4-chlorobenzoyl chloride, 3,5-dichlorobenzoic acid, 3,5-dichlorobenzoyl chloride, 2,6-dichlorobenzoic acid, 2,6-dichlorobenzoyl chloride, 4-nitrobenzoic acid, 4-nitrobenzoyl chloride 3,5-dinitrobenzoic acid, 3,5-dinitrobenzoyl chloride propylbenzoate, phenylbenzoate, and hexylbenzoate were purchased from Sigma-Aldrich Quimica SA., the remaining esters were obtained from in-house library or were prepared by the general procedure described below. Middlebrook 7H10 agar was purchased from Difco. Microwell tissue culture plates were purchased from Nunc. They then were prepared in stock solutions of 8 mg/mL in dimethyl sulfoxide (DMSO), and further diluted in Middlebrook 7H9 medium containing oleic acid, albumin, dextrose, and catalase (OADC; Sigma-Aldrich).

### Synthesis

### Acyl chloride synthesis

A solution of the chosen benzoic acid derivative in thionyl chloride (3 mL per mmol of acid) was refluxed for 5h, leading to the formation of the desired acyl chloride. The excess thionyl chloride is then removed by low pressure evaporation. The product is used without further purification.

### Ester synthesis

The desired benzoic acid derivative was dissolved in the desired alcohol (25 eq.) and placed under stirring. Then, thionyl chloride (1.5 eq) or sulfuric acid (0.5 eq) were added dropwise and the reaction was heated for 5 h at 50°C or 24h at 120°C to form the respective esters. The reaction was followed by thin layer chromatography (TLC) (toluene as eluent) until the reaction was complete. After, water is added and the ester is extracted with dichloromethane (DCM). The organic solution is then washed with saturated sodium bicarbonate solution, dried (Na2SO4) and evaporated. The ester was then purified by column chromatography (silica gel 60) using toluene as eluent.
- **Butyl benzoate (1a) ¹H RMN (300 MHz, Chloroform-d) δ** 8.11 - 8.02 (m, 2H), 7.62 - 7.46 (m, 2H), 7.46 - 7.39 (m, 1H), 4.35 (t, J = 6.6 Hz, 2H), 1.85 - 1.70 (m, 2H), 1.62 - 1.32 (m, 2H), 1.00 (t, J = 7.4 Hz, 3H). ¹³C **RMN (300 MHz, Chloroform-d) δ** 166.85 (C7), 132.77 (C4), 130.64 (C1), 129.53 (C2 e C6), 128.22 (C3 e C5), 65.02 (C9), 30.76 (C10), 19.27 (C11), 13.75 (C12).
- **Hexyl benzoate (1b) ¹H RMN (300 MHz, Chloroform-d) δ** 8.11 - 8.01 (m, 2H), 7.62 - 7.53 (m, 2H), 7.67 - 7.53 (m, 1H), 4.33 (t, J = 6.7 Hz, 2H), 1.75 (m, 2H), 1.48 -1.24 (m, 13H), 0.92 (t, J = 7.4 Hz, 3H). **¹³C RMN (300 MHz, Chloroform-d) δ** 166.78 (C7), 132.76 (C4), 130.56 (C1), 129.53 (C2 e C6), 128.30 (C3 e C5), 65.34 (C9), 31.56 (C10), 25.69 (C11-C13), 13.99 (C14).
- **Octyl benzoate (1c) ¹H NMR (300 MHz, Chloroform-d) δ** 8.11 - 8.02 (m, 2H), 7.63 - 7.48 (m, 1H), 7.48 - 7.39 (m, 2H), 4.33 (t, J = 6.7 Hz, 2H), 1.86 -1.71 (m, 2H), 1.52 - 1.40 (m, 2H), 1.38 -1.26 (m, 8H), 0.90 (t, J = 7.4 Hz 3H). ¹³C **RMN (300 MHz, Chloroform-d) δ** 166.85 (C7), 132.76 (C4), 130.70 (C1), 129.53 (C2 e C6), 128.30 (C3 e C5), 65.14 (C9), 31.71 (C10), 26.11 (C11-C15), 14.07 (C16).
- **Dodecyl benzoate (1e) ¹H NMR (300 MHz, Chloroform-d) δ** 8.11 - 8.01 (m, 2H), 7.62 - 7.53 (m, 1H), 7.53 - 7.39 (m, 2H), 4.33 (t, J = 6.7 Hz, 3H), 1.84 - 1.71 (m, 2H), 1.36 - 1.25 (m, 24H), 0.88 (t, J = 7.4 3H). ¹³C **RMN (300 MHz, Chloroform-d) δ** 166.88 (C7), 132.76 (C4), 130.71 (C1), 129.53 (C2 e C6), 128.30 (C3 e C5), 65.34 (C9), 31.17 (C10), 26 (C11-C19), 14.10 (C20).
- **Octyl 4-nitrobenzoate (2c) ¹H NMR (300 MHz, Chloroform-d) δ** 8.36-8.26 (ddd, Jₒ = 9 e Jₘ = 1,8; 2,1 Hz, 2H, C3-H e C5-H), 8.26 - 8.17 (ddd, Jₒ = 9 e Jₘ = 1,8; 2,1 Hz, 2H, C2-H e C6-H), 4.38 (t, J = 6.7 Hz, 1H), 1.88 -1.73 (m, 1H), 1.40 - 1.26 (m, 8H), 0.90 (t, J = 7.4 Hz, 3H). ¹³C **RMN (300 MHz, Chloroform-d) δ** 164.92 (C7), 150.49 (C4), 136.21 (C1), 130.64 (C2 e C6), 123.50 (C3 e C5), 66.24 (C9), 32 (C10), 25.80 (C11-C15), 14.06 (C16).
- **Decyl 3,5-dinitrobenzoate (3d) ¹H NMR (300 MHz, Chloroform-d) δ ¹H** NMR (300 MHz, Chloroform-d) **δ** 9.24 (t, J = 2.2 Hz, 1H), 9.17 (d, J = 2.1 Hz, 2H), 4.47 (t, J = 6.8 Hz, 2H), 1.61 (m, 2H), 1.52 -1.24 (m, 14H), 0.95 - 0.84 (t, J = 7.4, 3H). ¹³C **RMN (300 MHz, Chloroform-d) δ** 162.53 (C7), 122.26 (C4), 134.13 (C1), 129.38 (C2 e C6), 148.84 (C3 e C5), 67.30 (C9), 31.81 (C10), 25.51 (C11-C17), 14.08 (C18).
- **Hexadecyl 3,5-dinitrobenzoate (3g) ¹H NMR (300 MHz, Chloroform-d) δ** 9.24 (t, J = 2.1 Hz, 1H), 9.17 (d, J = 2.1 Hz, 2H), 4.47 (t, J = 6.8 Hz, 2H), 1.85 (m, 2H), 1.27 (m, 26H), 0.89 (t, J = 7.4, 3H). **¹³C RMN (300 MHz, Chloroform-d) δ** 162.66 (C7), 122.27 (C4), 134.18 (C1), 129.39 (C2 e C6), 148.74 (C3 e C5), 67.12 (C9), 31.96 (C10), 25.92 (C11-C23), 14.10 (C24).
- **Butyl 3-nitro-5-(trifluoromethyl)benzoate (4a) ¹H NMR (300 MHz, Chloroform-d) δ** 9.05 (dd, J = 1.9; 2.0 Hz, 1H), 8.69 (dd, J = 2.3; 1.9 Hz, 1H), 8.63 (dd, J = 2.3; 2.0 Hz, 1H), 4.45 (t, J = 6.7 Hz, 2H), 1.91-1.75 (m, 2H), 1.60 - 1.34 (m, 2H) 0.98 (t, J = 7.4, 3H).
- **Hexyl 3-nitro-5-(trifluoromethyl)benzoate (4b) ¹H NMR (300 MHz, Chloroform-d) δ** 9.05 (dd, J = 1.9; 2.1 Hz, 1H), 8.69 (dd, J = 2.1; 1.7 Hz, 1H), 8.63 (dd, J = 1.7; 2.0 Hz, 1H), 4.44 (t, J = 6.8 Hz, 2H), 1.84 (m, 2H), 1.55 - 1.25 (m, 6H) 0.93 (t, J = 7.4, 3H). **¹³C RMN (300 MHz, Chloroform-d) δ** 163.50 (C7), 120.61 (C4), 131.25 (C1), 127.57 (C2), 124.42 (C6), 148.84 (C3),133.85 (C5), 66.84 (C9), 31.38(C10), 26 (C11-C13), 14.05 (C14).
- **Octyl 3-nitro-5-(trifluoromethyl)benzoate (4c) ¹H NMR (300 MHz, Chloroform-d) δ** 9.05 (dd, J = 1.9; 2.0 Hz, 1H), 8.69 (dd, J = 2.1; 2.0 Hz, 1H), 8.63 (dd, J = 1.7; 1.8 Hz, 1H), 4.44 (t, J = 6.8 Hz, 2H), 1.84 (m, 2H), 1.31 (m, 10H), 0.89 (t, J = 7.4, 3H). ¹³C **RMN (300 MHz, Chloroform-d) δ** 163.20 (C7), 124.34 (C4), 133.29 (C1), 131.75 (C2), 127.71 (C6), 148.86 (C3),133.64 (C5), 66.76 (C9), 31.84(C10), 25.91 (C11-C15), 14.03 (C16).
- **Decyl 3-nitro-5-(trifluoromethyl)benzoate (4d) ¹H NMR (300 MHz, Chloroform-d) δ** 9.05 (dd, J = 1.9; 2.0 Hz, 1H), 8.68 (dd, J = 2.2; 2.1 Hz, 1H), 8.63 (dd, J = 1.7; 1.8 Hz, 1H), 4.44 (t, J = 6.8 Hz, 2H), 1.91 -1.76 (m, 2H), 1.36 - 1.24 (m, 10H), 0.95 - 0.82 (t, J = 7.4, 3H). ¹³C **RMN (300 MHz, Chloroform-d) δ** 163.45 (C7), 124.40 (C4), 133.19 (C1), 132.69 (C2), 127.45 (C6), 148.74 (C3),133.83 (C5), 66.82 (C9), 31.66 (C10), 25.91 (C11-C17), 14.06 (C18).
- **Dodecyl 3-nitro-5-(trifluoromethyl)benzoate (4e) ¹H NMR (300 MHz, Chloroform-d) δ** 9.05 (dd, J = 1.8; 1.9 Hz, 1H), 8.69 (dd, J = 2.0; 1.9 Hz, 1H), 8.63 (dd, J = 1.6; 1.8 Hz, 1H), 4.44 (t, J = 6.8 Hz, 2H), 1.91 - 1.76 (m, 2H), 1.29 (m, 20H), 0.95 - 0.84 (t, J = 7.4, 3H). **¹³C RMN (300 MHz, Chloroform-d)** δ 163.18 (C7), 120.70 (C4), 132.13 (C1), 127.35 (C2), 124.59 (C6), 148.77 (C3),133.68 (C5), 67.02 (C9), 31.88 (C10), 25.93 (C11-C19), 14.07 (C20).

### Amide synthesis

To a solution of the appropriate acyl chloride in dichloromethane, the desired amine (3 equivalents) was added. The reaction was followed by thin layer chromatography (TLC) (hexane:ethyl acetate, 7:3) until completion. Then, the solvent is evaporated and the crude is purified by column chromatography (silica gel 60) using hexane and ethyl acetate (7:3) as eluent, yielding the desired amide.
Alternatively, the desired benzoic acid derivative was dissolved in DCM and the desired amine (3 eq.) was added. Then, thionyl chloride (1.5 eq) was added dropwise and the reaction was heated for 5 h at 50°C to form the respective amides. The reaction was followed by thin layer chromatography (TLC) (hexane:ethyl acetate, 7:3) until the reaction was complete. After, water is added and the ester is extracted with dichloromethane (DCM). The organic solution is then washed with saturated sodium bicarbonate solution, dried (Na2SO4) and evaporated. The ester was then purified by column chromatography (silica gel 60) using hexane and ethyl acetate (7:3) as eluent.

**N-butyl-benzamide (5a) ¹H NMR (300 MHz, Chloroform-d) δ** 7.82 - 7.72 (m, 2H), 7.56 - 7.46 (m, 1H), 7.46 - 7.37 (m, 2H), 6.15 (s, 1H), 3.47 (td, J = 7.1, 5.7 Hz, 2H), 1.68 - 1.54 (m, 2H), 1.52 - 1.34 (m, 2H), 0.98 (t, J = 7.3 Hz, 3H). **¹³C RMN (300 MHz, Chloroform-d)** δ 167.74 (C7), 131.23 (C4), 134.95 (C1), 128.46 (C2 e C6), 126.89 (C3 e C5), 40.25 (C9), 31.82 (C10), 20.24 (C11), 13.77 (C12).

**N-hexyl-benzamide (5b) ¹H NMR (300 MHz, Chloroform-d) δ** 7.83 - 7.72 (m, 2H), 7.57 - 7.44 (m, 2H), 7.44 - 7.38 (m, 1H), 6.10 (s, 1H), 3.47 (td, J = 7.1, 5.7 Hz, 2H), 1.71 - 1.56 (m, 2H), 1.52 - 1.34 (m, 6H), 0.98 (t, J = 7.3 Hz, 3H). **¹³C RMN (300 MHz, Chloroform-d)** δ 167.63 (C7), 131.24 (C4), 134.84 (C1), 128.49 (C2 e C6), 126.85 (C3 e C5), 40.31 (C9), 31.73 (C10), 26.59 (C11-C13), 14 (C14).

**N-octyl-benzamide (5c) ¹H NMR (300 MHz, Chloroform-d) δ** 7.82 - 7.72 (m, 2H), 7.57 - 7.47 (m, 1H), 7.47 - 7.38 (m, 2H), 6.11 (s, 1H), 3.47 (td, J = 7.2, 5.6 Hz, 2H), 1.62 (m, 2H), 1.42 - 1.24 (m, 10H), 0.96 - 0.84 (t, J = 7.3 Hz, 3H). **¹³C RMN (300 MHz, Chloroform-d)** δ 167.52 (C7), 131.24 (C4), 134.92 (C1), 128.49 (C2 e C6), 126.86 (C3 e C5), 40.26 (C9), 32.03 (C10), 27.10 (C11-C15), 14.07 (C16).

**N-dodecyl-benzamide (5e) ¹H NMR (300 MHz, Chloroform-d) δ** 7.82 - 7.72 (m, 2H), 7.57 - 7.47 (m, 1H), 7.47 - 7.38 (m, 2H), 6.11 (s, 1H), 3.47 (td, J = 7.2, 5.7 Hz, 2H), 1.71 - 1.55 (m, 2H), 1.45 - 1.28 (m, 11H), 1.28 (m, 7H), 0.95 - 0.84 (t, J = 7.3 Hz, 3H). **¹³C RMN (300 MHz, Chloroform-d)** δ 167.49 (C7), 131.25 (C4), 134.92 (C1), 128.51 (C2 e C6), 126.84 (C3 e C5), 40.26 (C9), 31.88 (C10), 27.03 (C11-C19), 14.10 (C20).

**N-butyl-4-nitrobenzamide (6a)** ¹**H NMR (300 MHz, Chloroform-d) δ** 8.34 - 8.25 (ddd, Jₒ = 9 e Jₘ = 1,8; 2,1 Hz, 2H, C3-H e C5-H), 7.98 - 7.88 (ddd, Jₒ = 9 e Jₘ = 1,8; 2,1 Hz, 2H, C2-H e C6-H), 6.19 (s, 1H), 3.50 (td, J = 7.2, 5.7 Hz, 2H), 1.66 (m, 2H), 1.43 (m 2H), 0.99 (t, J = 7.3 Hz, 3H). **¹³C RMN (300 MHz, Chloroform-d)** δ 165.60 (C7), 149.44 (C4), 140.55 (C1), 128.09 (C2 e C6), 123.74 (C3 e C5), 40.31 (C9), 31.94 (C10), 20.01 (C11), 13.72 (C12).

**N-hexyl-4-nitrobenzamide (6b)** ¹**H NMR (300 MHz, Chloroform-d) δ** 8.36 - 8.25 (ddd, Jₒ = 9 e Jₘ = 1,8; 2,1 Hz, 2H, C3-H e C5-H), 7.99 - 7.88 (ddd, Jₒ = 9 e Jₘ = 1,8; 2,1 Hz, 2H, C2-H e C6-H), 6.14 (s, 1H), 3.50 (td, J = 7.2, 5.7 Hz, 2H), 1.66 (m, 2H), 1.49 - 1.24 (m, 6H), 0.99 (t, J = 7.3 Hz, 3H). **¹³C RMN (300 MHz, Chloroform-d)** δ 165.46 (C7), 149.60 (C4), 140.49 (C1), 128.07 (C2 e C6), 123.77 (C3 e C5), 40.51 (C9), 31.31 (C10), 26.65 (C11-C13), 13.98 (C14).

**N-octyl-4-nitrobenzamide (6c)** ¹**H NMR (300 MHz, Chloroform-d) δ** 8.27 - 8.16 (ddd, Jₒ = 9 e Jₘ = 1,8; 2,1 Hz, 2H, C3-H e C5-H), 7.90 - 7.80 (ddd, Jₒ = 9 e Jₘ = 1,8; 2,1 Hz, 2H, C2-H e C6-H), 6.10 (s, 1H), 3.41 (td, J = 7.2, 5.7 Hz, 2H), 1.37 - 1.15 (m, 12H), 0.87 - 0.76 (t, J = 7.3 Hz, 3H). **¹³C RMN (300 MHz, Chloroform-d)** δ 165.54 (C7), 149.56 (C4), 140.47 (C1), 128.08 (C2 e C6), 123.76 (C3 e C5), 40.68 (C9), 31.64 (C10), 26.84 (C11-C15), 14.05 (C16).

**N- dodecyl-4-nitrobenzamide (6e)** ¹**H NMR (300 MHz, Chloroform-d) δ** 8.35 - 8.25 (ddd, Jₒ = 9 e Jₘ = 1,8; 2,1 Hz, 2H, C3-H e C5-H), 7.98 - 7.88 (ddd, Jₒ = 9 e Jₘ = 1,8; 2,1 Hz, 2H, C2-H e C6-H), 6.17 (s, 1H), 3.49 (td, J = 7.2, 5.7 Hz, 2H), 1.67 (m, 4H), 1.28 (m, 16H), 0.95 - 0.82 (t, J = 7.3 Hz, 3H).

**N-butyl-3.5-dinitrobenzamide (7a)** ¹**H NMR (300 MHz, Chloroform-d) δ** 9.18 (t, J = 2.1 Hz, 1H), 8.96 (d, J = 2.1 Hz, 2H), 6.40 (s, 1H), 3.56 (td, J = 7.3, 5.7 Hz, 2H), 1.77 - 1.61 (m, 2H), 1.56 - 1.37 (m, 2H), 1.01 (t, J = 7.3 Hz, 3H). **¹³C RMN (300 MHz, Chloroform-d)** δ 162.93 (C7), 120.95 (C4), 138.36 (C1), 127.21 (C2 e C6), 148.55 (C3 e C5), 40.72 (C9), 31.63 (C10), 19.95 (C11), 13.70 (C12).

**N-hexyl-3.5-dinitrobenzamide (7b)** ¹**H NMR (300 MHz, Chloroform-d) δ** 9.17 (t, J = 2.1 Hz, 1H), 8.96 (d, J = 2.1 Hz, 2H), 6.42 (s, 1H), 3.55 (td, J = 7.3, 5.7 Hz, 2H), 1.77 - 1.64 (m, 2H), 1.37 - 1.33 (m, 6H), 0.98 - 0.87 (t, J = 7.3 Hz, 3H). **¹³C RMN (300 MHz, Chloroform-d)** δ 162.96 (C7), 120.95 (C4), 138.35 (C1), 127.17 (C2 e C6), 148.87 (C3 e C5), 40.89 (C9), 31.36 (C10), 26.79 (C11-C13), 13.98 (C14).

**N-octyl-3.5-dinitrobenzamide (7c)** ¹**H NMR (300 MHz, Chloroform-d) δ** 9.18 (t, J = 2.1 Hz, 1H), 8.95 (d, J = 2.1 Hz, 2H), 6.37 (s, 1H), 3.54 (td, J = 7.3, 5.7 Hz, 2H), 1.71 (m, 2H), 1.30 (dq, J = 7.9, 4.3 Hz, 10H), 0.95 - 0.85 (t, J = 7.3 Hz, 3H). **¹³C RMN (300 MHz, Chloroform-d)** δ 162.68 (C7), 120.95 (C4), 138.16 (C1), 127.17 (C2 e C6), 148.87 (C3 e C5), 40.90 (C9), 31.83 (C10), 26.90 (C11-C15), 14.05 (C16).

**N-decyl-3.5-dinitrobenzamide (7d)** ¹**H NMR (300 MHz, Chloroform-d) δ** 9.18 (t, J = 2.0 Hz, 1H), 8.96 (d, J = 2.1 Hz, 2H), 6.39 (s, 1H), 3.54 (td, J = 7.3, 5.7 Hz, 2H), 1.69 (m, 2H), 1.41 - 1.24 (m, 14H), 0.95 - 0.84 (t, J = 7.3 Hz, 3H). **¹³C RMN (300 MHz, Chloroform-d)** δ 162.98 (C7), 120.94 (C4), 138.23 (C1), 127.18 (C2 e C6), 148.68 (C3 e C5), 41.05 (C9), 31.93 (C10), 27.05 (C11-C17), 14.08 (C18).

**N-dodecyl-3.5-dinitrobenzamide (7e)** ¹**H NMR (300 MHz, Chloroform-d) δ** 9.18 (t, J = 2.1 Hz, 1H), 8.96 (d, J = 2.1 Hz, 2H), 6.40 (s, 1H), 3.54 (td, J = 7.3, 5.7 Hz, 2H), 1.69 (m, 2H), 1.38 (m, 2H), 1.29 (m, 16H), 0.96 - 0.84 (t, J = 7.3 Hz, 3H). **¹³C RMN (300 MHz, Chloroform-d)** δ 162.74 (C7), 120.94 (C4), 138.39 (C1), 127.16 (C2 e C6), 148.88 (C3 e C5), 40.96 (C9), 31.94 (C10), 26.91 (C11-C19), 14.09 (C20).

**N- tetradecyl-3.5-dinitrobenzamide (7f)** ¹**H NMR (300 MHz, Chloroform-d) δ** 9.14 (t, J = 2.0 Hz, 1H), 9.01 (d, J = 2.0 Hz, 2H), 7.08 (s, 1H), 3.58 - 3.45 (td, J = 7.3, 5.7 Hz, 2H), 1.76 (m, 2H), 1.65 (m, 2H), 1.35 (m, 2H), 1.29 - 1.22 (m, 18H), 0.93 - 0.82 (t, J = 7.3 Hz, 3H). **¹³C RMN (300 MHz, Chloroform-d)** δ 162.94 (C7), 121.25 (C4), 138.56 (C1), 127.50 (C2 e C6), 148.92 (C3 e C5), 41.39 (C9), 32.10 (C10), 27.32 (C11-C21), 14.36 (C22).

**N-hexadecyl -3.5-dinitrobenzamide (7g)** ¹**H NMR (300 MHz, Chloroform-d) δ** 9.18 (t, J = 2.1 Hz, 1H), 8.95 (d, J = 2.1 Hz, 2H), 6.36 (s, 1H), 3.54 (td, J = 7.3, 5.7 Hz, 2H), 1.69 (m, 3H), 1.39 (m, 3H), 1.33 - 1.28 (m, 6H), 1.27 (s, 16H), 0.95 - 0.84 (J = 7.3 Hz, 3H). **¹³C RMN (300 MHz, Chloroform-d)** δ 162.90 (C7), 120.95 (C4), 138.21 (C1), 127.14 (C2 e C6), 148.85 (C3 e C5), 40.93 (C9), 32.05 (C10), 26.93 (C11-C23), 14.10 (C24).

**N-butyl-3-nitro-5-(trifluoromethyl)-benzamide (8a)** ¹**H NMR (300 MHz, Chloroform-d) δ** 8.68 (dd, J = 1.9, 1.9 Hz, 1H), 8.57 (dd, J = 2.0, 1.9 Hz, 1H), 8.34 (dd, J = 1.7, 1.9 Hz, 1H), 6.25 (s, 1H), 3.45 (td, J = 7.2, 5.7 Hz, 2H), 1.59 (m, 2H), 1.46 - 1.28 (m, 2H), 0.91 (t, J = 7.3 Hz, 3H). **¹³C RMN (300 MHz, Chloroform-d)** δ 164.15 (C7), 120.70 (C4), 130.13 (C1), 125.05 (C2), 123.03 (C6), 148.55 (C3), 137.94 (C5), 40.41 (C9), 31.72 (C10), 26.08 (C11), 13.80 (C12).

**N-hexyl-3-nitro-5-(trifluoromethyl)-benzamide (8b)** ¹**H NMR (300 MHz, Chloroform-d) δ** 8.76 (dd, J = 1.9, 1.9 Hz, 1H), 8.63 (dd, J = 2.0, 1.9 Hz, 1H), 8.42 (dd, J = 1.7, 1.9 Hz, 1H), 6.25 (s, 1H), 3.53 (td, J = 7.3, 5.7 Hz, 2H), 1.68 (m, 2H), 1.48 - 1.32 (m, 6H), 0.91 (t, J = 7.3 Hz, 3H). **¹³C RMN (300 MHz, Chloroform-d)** δ 163,72 (C7), 120.90 (C4), 129,95 (C1), 124,68 (C2), 122,82 (C6), 148.32 (C3), 137.85 (C5), 40.65 (C9), 31.37 (C10), 26.76 (C11-C13), 13.95 (C14).

**N-octyl-3-nitro-5-(trifluoromethyl)-benzamide (8c)** ¹**H NMR (300 MHz, Chloroform-d) δ** 8.76 (dd, J = 1.9, 1.9 Hz, 1H), 8.63 (dd, J = 2.0. 1.9 Hz, 1H), 8.42 (dd, J = 1.6, 1.9 Hz, 1H), 6.27 (s, 1H), 3.53 (td, J = 7.3, 5.7 Hz, 2H), 1.67 (m, 2H), 1.41 - 1.25 (m, 10H), 0.91 (t, J = 7.3 Hz, 3H). **¹³C RMN (300 MHz, Chloroform-d)** δ 163,66 (C7), 122.44 (C4), 129,95 (C1), 124,68 (C2), 122,82 (C6), 148.32 (C3), 137.85 (C5), 40.87 (C9), 31.63 (C10), 29.48 (C11-C15), 14.03 (C16).

**N-decyl-3-nitro-5-(trifluoromethyl)-benzamide (8d)** ¹**H NMR (300 MHz, Chloroform-d) δ** 8.77 (dd, J = 1.9, 1.9 Hz, 1H), 8.63 (dd, J = 2.0, 1.9 Hz, 1H), 8.42 (dd, J = 1.7, 1.9 Hz, 1H), 6.30 (s, 1H), 3.52 (td, J = 7.3, 5.7 Hz, 2H), 1.69 (m, 2H), 1.39 (m, 4H), 1.34 - 1.24 (m, 10H), 0.91 (t, J = 7.3 Hz, 3H). **¹³C RMN (300 MHz, Chloroform-d)** δ 163,91 (C7), 122.99 (C4), 132.91 (C1), 129.97 (C2), 124.75 (C6), 148.41 (C3), 137.61 (C5), 40.87 (C9), 31.73 (C10), 29.41 (C11-C17), 14.03 (C18).

**N-dodecyl-3-nitro-5-(trifluoromethyl)-benzamide (8e)** ¹**H NMR (300 MHz, Chloroform-d) δ** 8.76 (dd, J = 1.8, 1.9 Hz, 1H), 8.63 (dd, J = 2.0, 1.9 Hz, 1H), 8.42 (dd, J = 1.7, 1.9 Hz, 1H), 6.29 (s, 1H), 3.52 (td, J = 7.3, 5.7 Hz, 2H), 1.76 - 1.63 (m, 2H), 1.38 (m, 4H), 1.29 (m, 14H), 0.91 (t, J = 7.3 Hz, 3H). **¹³C RMN (300 MHz, Chloroform-d)** δ 163,78 (C7), 122.99 (C4), 132.91 (C1), 129.97 (C2), 124.75 (C6), 148.47 (C3), 137.80 (C5), 40.85 (C9), 31.38 (C10), 29.57 (C11-C19), 13.46 (C20).

### Bacterial strains and culture conditions

*Mycobacterium tuberculosis* H37Rv (ATCC 27294) were cultivated in Middlebrook 7H9 medium supplemented with OADC and 0.05 % tyloxapol (Sigma-Aldrich) and incubated at 37 ºC until exponential growth phase was achieved.

### Plasma stability

Pooled human plasma (960 µL), pH 7.4 phosphate buffered saline (216 µL), 18 µL ACN and 6 µL of a 10-1 M stock solution of the prodrug in ACN were mixed in a 2mL vial. The suspensions were incubated at 37 °C and 50 µL aliquots were removed, mixed with 450 µL of ACN: ZnSO4 1% 1:1 and centrifuged. The supernatant was removed and analysed by HPLC, the remaining substrate and the correspondent acid measured.

### Buffer stability

1176 µL pH 7.4 phosphate buffer or pH 5.9 phosphate buffer, 18 µL ACN and 6 µL of a 10-1 M stock solution of the prodrug in ACN were mixed in a 2mL vial. The solution was incubated at 37 °C and 50 µL aliquots were removed, mixed with 450 µL of ACN: H2O 1:1. The solution was analysed by HPLC, the remaining substrate and the correspondent acid were both measured.

### HPLC analysis

Two HPLC systems were used. The buffer and plasma stability studies were performed in an HPLC system with a photodiode detector L-3000 Photo Diode Array Detector, Merck-Hitachi L-6000 pump, Merck-Hitachi D-2500 integrator, and Merck RP-8 column. Activation studies were performed in an HPLC system with a UV detector Merck-Hitachi UV-L7400, Merck Hitachi L-7100 pump, an auto sampler Merck-Hitachi AS 2000, and a Merck-Hitachi D-2500 integrator and Merck RP-8 column. The eluant was a mixture of acetonitrile (60%/70%) and aqueous phosphate buffer with 5% of KH2PO4/H3PO4 0.025M (40%/30%). The flow rate was always 1 mL/1 min and wavelength was set at 230 nm. All quantifications were evaluated using calibration curves from stock solutions.

### Determination of the Minimum Inhibitory Concentration (MIC) and Minimum Bactericidal Concentrations (MBC)

The MICs were determined by the broth microdilution method in 96-well plates. Briefly, M. *tuberculosis* bacterial cultures in exponential growth phase were collected by centrifugation, washed in PBS and re-suspended in fresh culture medium. Clumps of bacteria were removed by ultrasonic treatment of the bacteria suspension in an ultrasonic water bath for 5 min followed by a low-speed centrifugation (500 x g) for 2 min. Single cell suspension was verified by microscopy. The microplates containing a bacterial suspension corresponding to approximately 105 colony-forming units per ml were incubated with the selected concentrations of the compounds. Every other day, the optical density of the wells was measured in a Tecan M200 spectrophotometer, following 30 s of orbital agitation. These values were used to produce the growth curves. At the 10th day of incubation, the MIC was determined, corresponding to the concentration with no visible turbidity. Optical density measures were taken until the 15th day of incubation following which the bacterial samples were recovered from the MIC test microplates and plated in 7H10 + OADC solid medium. The MBC was determined following 3 weeks of incubation, corresponding to the concentration of compound that produced no colonies on the solid medium. Bacteria treated with DMSO solvent at the same proportions as present during the compound tests were used as a control. Isoniazid was used as a positive control for bacteria killing and assay validation following EUCAST guidelines.

### Mycobacterial homogenate

A crude whole mycobacterial homogenate was prepared according to [19]. A culture of exponentially growing M. *smegmatis* ATCC607 variant mc2 155 with an O.D.600 nm of 0.8-1.0 was harvested by centrifugation at T = 4 °C for 10 min, washed and re-suspended in pH = 7.4 phosphate buffer saline PBS (25 mL for each 750 mL of the initial growing broth). The bacterial homogenate was prepared using an ultra-sound probe with a sequence of five cycles of 2 min each. The homogenate was afterwards divided in 1 mL portions and kept at -80 °C until use. Total protein concentration was 1.4 mg mL-1.

### Liposomal formulation

Liposomes were obtained by hydration of a solution containing the lipids and the compound to be incorporated, following the following steps:

The lipids used were dimyristoylphosphatidylcholine (DMPC) and dimyristoylphosphatidylglycerol (DMPG). The lipids and the prodrug were weighed, transferred to a round bottom flask, dissolved in dichloromethane and the organic solvent was evaporated at a rotary evaporator to form a lipid film. The flask was dried in a vacuum pump to remove any remaining solvent and 1 ml of isotonic phosphate buffer pH 7.4 (PBS) was added at a temperature at least 10°C higher than the phase transition temperature of the lipids present. The flask was then stirred for five minutes in order to hydrate the lipid film completely, waited a few minutes and stirred again for another five minutes after which the mixture was placed in an ultrasonic bath for 3 periods of 2 minutes, interspersed with 2 minutes rest. All prepared suspensions were observed under a phase-contrast optical microscope at 400x magnification.

To determine the incorporation efficiency (EE), an aliquot of the liposomal suspension obtained after hydration was taken and another aliquot of the liposomal suspension obtained after centrifugation, removal of the supernatant, and resuspension of the pellet in PBS in the original volume it had before centrifugation. The EEs were then calculated as the ratio of the concentration of the compound in the resuspended liposomal suspension/concentration of the compound in the initial liposomal suspension.

### Conditions of incubations and preparation of samples

The initial substrate concentration was 5 x 10-4 M in all stability assays. All incubations were carried out at pH 7.4 and 37 °C under agitation using the phosphate buffer described above as diluting agent. The levels of dilution of the mycobacterial homogenate (20%) and human plasma (80%) in the incubates were chosen following preliminary assays to ensure pseudo-first order kinetics in the hydrolysis of benzoates. Acetonitrile (2%) was used in all studies to ensure adequate solubilization of the substrates. The benzoates were added from 10 to 1M acetonitrile stock solutions. After incubation, aliquots of 50 µL were taken into vials containing 450 µL of a 1:1 solution of 1% zinc sulfate and acetonitrile, mixed in a vortex and centrifuged for 10 min at 15,000 rpm. The supernatant was then injected into the HPLC and analysed for quantification of benzoic acid and remaining benzoate. All quantifications were performed using calibration curves.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

Where ranges are provided, the range limits are included. Furthermore, it should be understood that unless otherwise indicated or otherwise evident from the context and/or understanding of a technical expert, the values which are expressed as ranges may assume any specific value within the ranges indicated in different achievements of the invention, at one tenth of the lower limit of the interval, unless the context clearly indicates the contrary. It should also be understood that, unless otherwise indicated or otherwise evident from the context and/or understanding of a technical expert, values expressed as range may assume any sub-range within the given range, where the limits of the sub-range are expressed with the same degree of precision as the tenth of the unit of the lower limit of the range.

The dependent claims further set out particular embodiments of the disclosure.

### References

1. Global Tuberculosis Report. WHO, Geneva 2021. ISBN: 9789240037021
2. Park M, Satta G, Kon OM. An update on multidrug-resistant tuberculosis. Clin Med (Lond). 2019 Mar;19(2):135-139. doi: 10.7861/clinmedicine.19-2-135
3. D. Pires et al., "Esters of pyrazinoic acid are active against pyrazinamide-resistant strains of Mycobacterium tuberculosis and other naturally resistant mycobacteria in vitro and ex vivo within macrophages," Antimicrob. Agents Chemother., vol. 59, no. 12, pp. 7693-7699, Dec. 2015, doi: 10.1128/AAC.00936-15.
4. M. F. Simões, E. Valente, M. J. R. Gómez, E. Anes, and L. Constantino, "Lipophilic pyrazinoic acid amide and ester prodrugs stability, activation and activity against M. tuberculosis.," Eur. J. Pharm. Sci., vol. 37, no. 3-4, pp. 257-63, Jun. 2009, doi: 10.1016/j.ejps.2009.02.012
5. P. Gu, L. Constantino, and Y. Zhang, "Enhancement of the antituberculosis activity of weak acids by inhibitors of energy metabolism but not by anaerobiosis suggests that weak acids act differently from the front-line tuberculosis drug pyrazinamide.," J. Med. Microbiol., vol. 57, no. Pt 9, pp. 1129-34, Sep. 2008, doi: 10.1099/jmm.0.2008/000786-0.
6. E. Valente, M. F. Simões, B. Testa, and L. Constantino, "Development of a method to investigate the hydrolysis of xenobiotic esters by a Mycobacterium smegmatis homogenate.," J. Microbiol. Methods, vol. 85, no. 2, pp. 98-102, May 2011, doi: 10.1016/j.mimet.2011.02.003.
7. Y. Zhang, H. Zhang, and Z. Sun, "Susceptibility of Mycobacterium tuberculosis to weak acids.," J. Antimicrob. Chemother., vol. 52, no. 1, pp. 56-60, 2003.
8. Caroline Shi-Yan Foo, Benoit Lechartier, Gaëlle S. Kolly, Stefanie Boy-Röttger, João Neres, Jan Rybniker, Andréanne Lupien,a Claudia Sala,a Jérémie Piton,a and Stewart T. Characterization of DprE1-Mediated Benzothiazinone Resistance in Mycobacterium tuberculosis. Antimicrob Agents Chemother. 2016 Nov; 60(11): 6451-6459. doi: 10.1128/AAC.01523-16
9. Giulia Manina, Marco Bellinzoni, Maria Rosalia Pasca, João Neres, Anna Milano, Ana Luisa De Jesus Lopes Ribeiro, Silvia Buroni, Henrieta Skovierová, Petronela Dianišková. Biological and structural characterization of the Mycobacterium smegmatis nitroreductase NfnB, and its role in benzothiazinone resistance. Mol Microbiol . 2010 Sep;77(5):1172-85. doi: 10.1111/j.1365-2958.2010.

## Claims

1. Compound of formula I or a pharmaceutically acceptable salt, or ester or solvate thereof 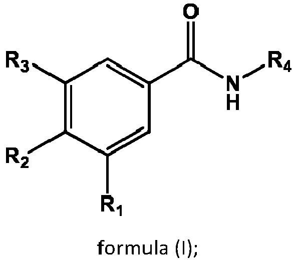
wherein R₁, R₂, R₃ and R₄ are independently selected from each other;
R₁, R₂ and R₃ are selected from NO₂, CF₃, OCF₃, F, Cl, Br, I, H, CN, OCH₃, CH₃;
R₄ is an alkyl, alkenyl or alkynyl chain with at least 3 carbons;
Provided that if R₁ and R₃ are NO₂ then R₄ is not a hexyl or hexadecanoyl chain.

2. Compound according to the previous claim wherein R₄ is an unsubstituted alkyl chain.

3. Compound according to any of the previous claims wherein R₄ is a C₇ chain; C₈ chain; C₉ chain; C₁₀ chain; C₁₁ chain, C₁₂ chain, C₁₃ chain, or C₁₄ chain.

4. Compound according to any of the previous claims for use in medicine or veterinary; preferably for use in any condition susceptible of being improved or prevented by selective inhibition of the enzyme DprE1.

5. Compound for use according to any of the previous claims for use in the treatment or prevention of a mycobacterial infection.

6. Compound for use according to any of the previous claims for use in the treatment or prevention of tuberculosis; preferably caused by an organism from the *Mycobacterium tuberculosis* Complex; preferably *Mycobacterium bovis* or *Mycobacterium tuberculosis.*

7. Compound for use according to the previous claims 4-5 wherein the mycobacterial infection is caused by a non-tuberculous mycobacteria (NTM) selected from the group consisting of: *Mycobacterium avium* complex (MAC), *Mycobacterium smegmatis, Mycobacterium gordonae, Mycobacterium kansasii, Mycobacterium terrae, Mycobacterium scrofulaceum, Mycobacterium vaccae, Mycobacterium marinum, Mycobacterium lentiflavum, Mycobacterium fortuitum, Mycobacterium chelonae, Mycobacterium abscessus, Mycobacterium intracellulare and Mycobacterium avium.*

8. Compound as described in any of the previous claims wherein R₁, R₂ and R₃ are independently select from NO₂, CF3 and H; preferably at least R₁ or R₂ or R₃ is NO₂.

9. Compound as described in any of the previous claims wherein R₁ and R₃ are NO₂ and R₄ is selected from: C₄ chain; C₈ chain; C₁₀ chain; C₁₂ chain; C₁₄ chain.

10. Compound as described in any of the previous claims wherein R₁ is CF₃ and R₃ is NO₂ and R₄ is selected from: C₄ chain; C₆ chain; C₈ chain; C₁₀ chain; C₁₂ chain.

11. Compound as described in any of the previous claims wherein R₂ is NO₂ and R₄ is selected from: C₄ chain; C₆ chain; C₈ chain; C₁₀ chain; C₁₂ chain.

12. Compound as described in any of the previous claims wherein the compound is:

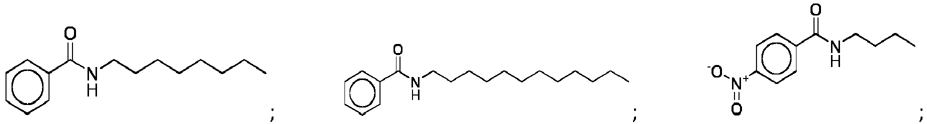

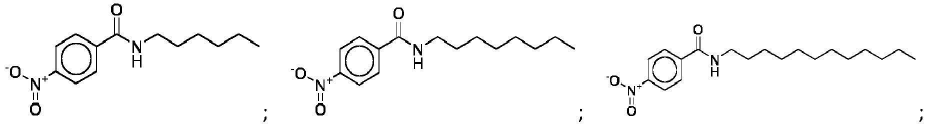

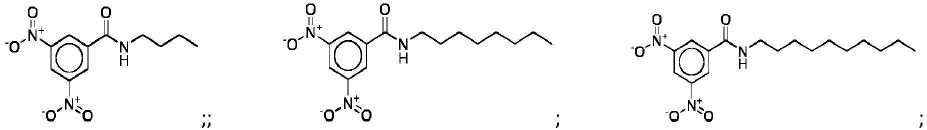

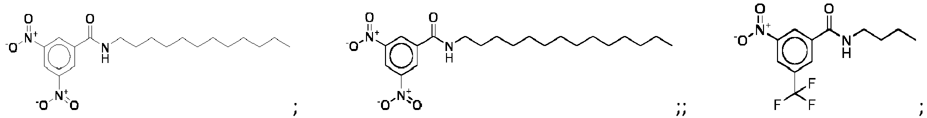

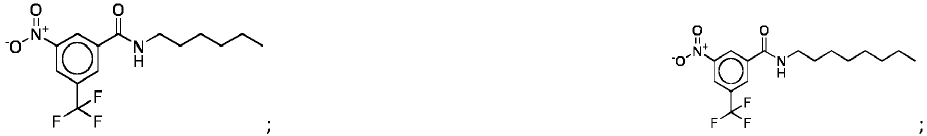

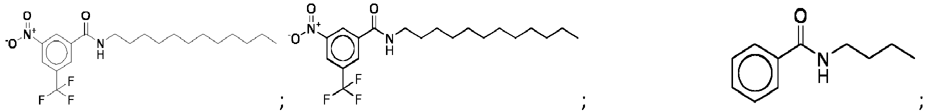

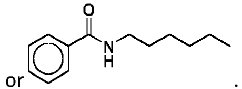

13. Compound as described in any of the previous claims for use in combination with at least one anti-HIV agent, wherein the anti-HIV agent is selected from a HIV protease inhibitor, a HIV nucleoside reverse transcriptase inhibitor, a HIV non-nucleoside reverse transcriptase inhibitor, or a HIV integrase inhibitor.

14. Compound as described in any of the previous claims for use in combination with at least a second tuberculosis drug, wherein the further tuberculosis drug is selected from the group of isoniazid, rifamycin and derivatives, pyrazinamide, ethambutol, cycloserine, ethionamide, streptomycin, amikacin, kanamycin, rifampin (rifampicin), aminoglycosides, capreomycin, p-aminosalicyclic acid, fluoroquinolones such as levofloxacin, moxafloxacin or gatifloxacin, or mixtures thereof.

15. Pharmaceutical composition comprising (i) a therapeutically effective amount of a compound as described in any of the previous claims or a pharmaceutically acceptable salt or solvate thereof; and (ii) a pharmaceutically acceptable excipient.
